# EUROPÄISCHE PATENTSCHRIFT

(11) **EP 4 392 141 B1**
(45) Veröffentlichungstag und Bekanntmachung des Hinweises auf die Patenterteilung: **03.09.2025**
(21) Anmeldenummer: 22751296.9
(22) Anmeldetag: 12.07.2022
(51) Int. Cl.: A61Q 5/06, A61K 8/58

(54) **VERFAHREN ZUM FÄRBEN VON KERATINISCHEM MATERIAL, INSBESONDERE MENSCHLICHEN HAAREN**
METHOD FOR DYEING KERATINOUS MATERIAL, IN PARTICULAR HUMAN HAIR
MÉTHODE DE COLORATION D'UNE MATIÈRE KÉRATINIQUE, EN PARTICULIER DES CHEVEUX HUMAINS

(30) Priorität: 26.08.2021 DE 102021209370
(43) Veröffentlichungstag der Anmeldung: 03.07.2024
(73) Patentinhaber: Henkel AG & Co. KGaA, 40589 Düsseldorf (DE)
(72) Erfinder: KRUPPA, Carolin, 40721 Hilden (DE); LECHNER, Torsten, 40764 Langenfeld (DE); NOWOTTNY, Marc, 41069 Mönchengladbach (DE); MATHIASZYK, Carsten, 45277 Essen (DE); WALTER, Andreas, 40880 Ratingen (DE); SCHOEPGENS, Juergen, 41366 Schwalmtal (DE); TAIRI, Avni, 47138 Duisburg (DE); JAISER, Phillip, 40764 Langenfeld (DE)
(86) Internationale Anmeldenummer: PCT/EP2022/069408
(87) Internationale Veröffentlichungsnummer: WO 2023/025452

(56) Entgegenhaltungen:
- WO-A1-2021/121723

## Beschreibung

Gegenstand der vorliegenden Anmeldung ist ein Verfahren zum Färben von keratinischem Material, insbesondere menschlichen Haaren, welches die Anwendung von zwei Mitteln (a) und (b) umfasst. Das Mittel (a) ist wasserarm bzw. wasserfrei und durch seinen Gehalt an mindestens einer organischen Siliciumverbindung und einer farbgebenden Verbindung gekennzeichnet. Das Mittel (a) wird zuerst auf das Keratinmaterial bzw. die Haare appliziert. Danach wird das wasserhaltige Mittel (b) auf das noch mit dem Mittel (a) beaufschlagte Keratinmaterial bzw. Haar gegeben, wobei das im Mittel (b) enthaltene Wasser bei Kontakt mit dem Mittel (a) zu einer gezielten Hydrolyse der Siliciumverbindungen führt. Nach dem Einwirkenlassen beider Mittel (a) und (b) werden diese dann gemeinsam ausgespült.

Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit Tensid-haltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbemitteln und Färbeverfahren. Besonders im Fokus stehen aktuell Färbesysteme, bei welchen die farbgebenden Verbindungen integriert in einen Film an die Oberfläche der Haarfaser angelagert werden, so dass diese auf Wunsch des Anwenders wieder rückstandslos und ohne Veränderung der ursrpünglichen Haarfarbe entfernt werden können.

EP 2168633 B1 beschäftigt sich mit der Aufgabenstellung, langanhaltende Haarfärbungen unter Einsatz von Pigmenten zu erzeugen. Die Schrift lehrt, dass sich bei Verwendung der Kombination aus einem Pigment, einer organischer Siliciumverbindung, einem filmbildenden Polymer und einem Lösungsmittel auf Haaren Färbungen erzeugen lassen, die gegenüber Abrieb und/oder Shampoonierungen besonders widerstandsfähig sind.

Der große Vorteil der in der EP 2168633 B1 eingsetzten Alkoxy-Silane liegt darin, dass die hohe Reaktivität dieser Verbindungsklasse ein sehr schnelles Coating ermöglicht. So können bereits nach sehr kurzen Anwendungszeiträumen von nur wenigen Minuten extrem gute Färbeergebnisse erzielt werden. Neben diesen Vorteilen birgt die hohe Reaktivität der Alkoxy-Silane jedoch auch einige Nachteile.

In WO 2021/121723 A1 wird ein Verfahren zum Färben von Keratinmaterial beschrieben, bei welchem die Mittel (a) und (b) auf das Keratinmaterial appliziert werden. Das Mittel (a) enthält mindestens ein Silan, einen Hydroxycarbonsäureester und ein Diol. Das Mittel (b) enthält ein Versiegelungsreagenz, und mindestens eines der Mittel (a) und (b) enthält ferner eine farbgebende Verbindung.

Die in den Färbungen eingesetzten organischen Siliciumverbindungen sind hochreaktive Verbindungen, die in Gegenwart von Wasser eine Hydrolyse oder Oligomerisierung und/oder Polymerisierung eingehen. Wichtig ist, die Geschwindigkeit der Oligomerisierung bzw. Polymerisierung so anzupassen, dass sie die Anwendung des Färbemittels in einem für die Anwender akzeptablen Zeitraum ermöglicht. Aufgrund ihrer hohen Reaktivität können die organischen Alkoxy-Silane nicht zusammen mit größeren Wassermengen konfektioniert werden, da ein großer Überschuss an Wasser die sofortige Hydrolyse und im Anschluss daran eine Polymerisation initiiert. Die bei Lagerung der Alkoxy-Silane in wässrigem Medium stattfindende Polymerisierung äußert sich in einer Verdickung oder Gelierung der wässrigen Zubereitung. Hierdurch werden die Zubereitungen so hochviskos, gelig bzw. gallertartig, dass sie nicht mehr gleichmäßig auf dem Keratinmaterial aufgetragen werden können. Zudem ist die Lagerung der Alkoxy-Silane in Gegenwart hoher Wassermengen mit einem Verlust ihrer Reaktivität verbunden, so dass auch die Ausbildung eines widerstandsfähigen und gleichmäßigen Coatings auf dem Keratinmaterial nicht mehr möglich ist. Insbesondere bei Färbeverfahren ist die Bildung eines gleichmäßigen Coatings jedoch besonders wichtig.

Aus diesen Gründen ist es notwendig, die organischen Alkoxy-Silane in wasserfreiem bzw. wasserarmem Milieu zu lagern und die entsprechenden Zubereitungen in einem separaten Container zu konfektionieren. Die wasserarmen Zubereitungen, welche die Alkoxy-Silane enthalten, können auch als "Silan-Blend" bezeichnet werden.

Für die Applikation auf dem Keratinmaterial muss der Anwender nun diesen Silan-Blend in eine anwendungsbereite Mischung überführen, so dass die Ausbildung des Coatings initiiert wird. Im Rahmen der zu dieser Erfindung führenden Arbeiten wurden die Silan-Blends mit verschiedenen Formulierungen abgemischt und diese Abmischungen im Hinblick auf ihre anwendungstechnischen Eigenschaften getestet. In einer Testreihe wurde aus dem Silan-Blend und einer wasserhaltigen Träger-Formulierung eine Anwendungsmischung mit hohem Wassergehalt erzeugt und diese auf Haarsträhnen appliziert. Bei dieser Form der Anwendung begann die Oligomerisierung bzw. Polymerisierung der Silane direkt bei Kontakt mit dem Wasser in der Anwendungsmischung, die sich jedoch noch nicht auf den Haaren befand. Es zeigte sich, dass das Farbergebnis stark von der Geübtheit des Anwenders und der Schnelligkeit abhing, mit der dieser die Anwendungsmischung auf das Haar auftrug. In der Folge waren - insbesondere bei langsamer Anwendung, ungeübten Erstanwendern oder großen Arealen der zur färbenden Haarpartien - die Haare sehr ungleichmäßig bzw. an bestimmten Stellen unzureichend gefärbt.

Demnach ist es nach wie vor eine große Herausforderung, die Polymerisationsgeschwindigkeit, d.h. die Geschwindigkeit, mit der sich das Coating auf dem Keratinmaterial ausbildet, optimal an die Anwendungsbedingungen anzupassen.

Es war die Aufgabe der vorliegenden Anmeldung, ein Verfahren zum Behandeln von keratinischem Material aufzufinden, mittels welchem die Polymerisationsgeschwindigkeit der organischen Alkoxy-silane an die Anwendungsbedingungen, insbesondere an die bei Anwendung auf dem menschlichen Kopf herrschenden Bedingungen, angepasst werden konnte. Mit anderen Worten wurde nach einem Verfahren gesucht, welches die reproduzierbare Ausbildung eines für Färbeverfahren geeigneten, gleichmäßig gefärbten Films erlaubt, der auf dem ganzen Kopf des Anwenders zu einer gleichmäßigen Färbung führt.

Überraschenderweise hat sich herausgestellt, dass diese Aufgabe in vollem Umfang gelöst werden kann, wenn das Keratinmaterial in einem Verfahren behandelt wird, bei dem zwei Mittel (a) und (b) sukzessive auf das Keratinmaterial bzw. das Haar appliziert werden. Bei dem Mittel (a) handelt es sich um eine wasserarme Formulierung, welche eine oder mehrere Alkoxy-Silane und zusätzlich mindestens eine farbgebende Verbindung enthält. Aufgrund des geringen Wassergehalts findet die Oligomerisierung bzw. Polymerisierung der Alkoxy-Silane im Mittel (a) nicht oder nur sehr begrenzt statt. Dieses wasserarme und farbstoffhaltige Mittel (a) wird nun auf das Keratinmaterial appliziert.

In einem Folgeschritt wird nun das Mittel (b) auf die Stellen des Keratinmaterials aufgetragen, auf welchen sich noch das Mittel (a) befindet. Das Mittel (b) enhält als wesentlichen Bestandteil eine definierte Menge Wasser. Mit dem Auftragen des Mittels (b) auf dem Keratinmaterial werden nun beide Mittel (a) und (b) miteinaner vermischt, was beispielsweise durch kräftiges Reiben oder Massieren des mit beiden Mitteln beaufschlagten Keratinmaterials unterstützt werden kann. Mit dem Auftrag des Mittels (b) kommen die im Mittel (a) befindlichen Alkoxy-Silane also mit einer definierten Menge Wasser in Kontakt, so dass die Oligomierisierung/Polymerisierung der Silane erst zu dem Zeitpunkt gestartet wird, an dem die Silane sich bereits an der Stelle auf dem Keratinmaterial bzw. den Haaren befinden, an der auch der gefärbte Film ausgebildet werden soll.

Ein erster Gegenstand der vorliegenden Erfindung ist ein Verfahren zum Färben von keratinischem Material, insbesondere menschlichen Haaren, umfassend die folgenden Schritte in der angegebenen Reihenfolge:
(1) Anwendung eines Mittels (a) auf dem keratinischem Material, wobei das Mittel (a) - bezogen auf das Gesamtgewicht des Mittels (a) - enthält:
   (a1) weniger als 10 Gew.-% Wasser,
   (a2) mindestens eine organische Siliciumverbindung aus der Gruppe der Silane mit einem, zwei oder drei Siliciumatomen, und
   (a3) mindestens ein farbgebende Verbindung,
(2) Anwendung eines Mittels (b) auf dem keratinischem Material, das noch mit dem Mittel (a) beaufschlagt ist, wobei das Mittel (b) enthält:
   (b1) Wasser
(3) Einwirkenlassen beider Mittel (a) und (b) auf das keratinische Material, und
(4) Ausspülen beider Mittel (a) und (b).

Es hat sich gezeigt, dass bei Anwendung dieses neuen Verfahrens auf menschlichen Haaren ein reproduzierbares und gleichmäßiges Farbergebnis erhalten werden konnte, das sich auch durch intensive Farbintensitäten und sehr gute Echtheiten auszeichnete. Von besonderem Vorteil war hierbei, dass das Farbergebnis nicht von dem Zeitraum abhängig war, den der Anwender für die Färbung benötigte.

### Keratinisches Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

### Färben von keratinischem Material

Der Begriff "Verfahren zur Färbung" betrifft im Rahmen dieser Erfindung all diejenigen Färbemethoden, bei welchen auf dem Keratinamterial ein gefärbter Film erzeugt wird. Die Färbung des Films kann hierbei durch all diejenigen farbgebenden Verbindungen zustande kommen, die kosmetisch geeignet sind, sich auf der Oberfläche der Keratinmaterialien ablagern und in diesen Film inkorporieren lassen. Bei solchen farbgebenden Verbindungen handelt es sich beispielsweise insbesondere um Pigmente und direktziehende Farbstoffe, ganz besonders bevorzugt um Pigmente.

Im Rahmen des beschriebenen Verfahrens werden die Mittel (a) und (b) nacheinander auf dem keratinischen Material, insbesondere den menschlichen Haaren, appliziert. Bei den Mitteln (a) und (b) handelt es sich jeweils um anwendungsbereite Mittel. Die beiden Mittel (a) und (b) sind voneinander verschieden.

### Schritt (1), Anwendung des Mittels (a) auf dem keratinischen Material

In Schritt (1) des erfindungsgemäßen Verfahrens wird das Mittel (a) auf dem keratinischen Material, insbesondere den menschlichen Haaren, angewendet.

Die Anwendung des Mittels (a) erfolgt hierbei zum Beispiel durch das Verteilen oder Verteilen und Einmassieren des Mittels (a) auf dem keratinischen Material (bzw. den Haaren) mit Hilfe der behandschuhten Hand, eines Pinsels, einer Applikatorflasche oder einer Aplicette. Das keratinische Material bzw. die Haare sind zu diesem Zeitpunkt bevorzugt handtuchtrocken oder trocken.

Trockenes keratinisches Material, insbesondere trockenes Haar, ist das Material, das direkt vor dem Färbeverfahren (d.h. bis zu drei Stunden vor dem Färbeverfahren) keiner zusätzlichen Behandlung unterzogen und auch nicht mit Wasser oder mit Wasser/Shampoo angefeuchtet wurde.

Als handtuchtrockenes keratinisches Material, inbesondere handtuchtrockenes Haar, wird das Material/Haar bezeichnet, das innerhalb von maximal innerhalb von 30 Minuten, bevorzugt 15 Minuten, vor Beginn des Färbeverfahrens mit Wasser benetzt bzw. gewaschen wurde, und im Anschluss daran mit einem Handtuch für ca. 30 Sekunden trocken gerubbelt wird.

Beispiel: Der Anwender macht sein Haar unter dem Wasserhahn vollständig nass, indem er es unter laufendem, auf ca. 35 °C temperiertes Wassser vollständig mit Wasser durchtränkt. Dann trocknet er das Haar für 30 Sekunden mit einem trockenen Handtuch ab.

Handtuchtrockenes Haar zeichnet sich dadurch aus, dass es nicht mehr tropfnass ist, d.h. auf der Haaroberfläche befinden sich keine signifikanten Wasseranteile mehr. Dennoch besitzt das Haar aufgrund der innerhalb der Haarfaser vorhandenen Wassermoleküle noch eine Restfeuchte, wodurch die Haarfasern gequollen sind. Die Anwendung des Mittels (a) auf handtuchtrockenem Keratinmaterial, insbesondere handtuchtrockenem Haar, ist ganz besonders bevorzugt. Die geringe Wassermenge, die bei handtuchtrockenem Haar vorhanden ist, erleichtert das Auftragen und Verteilen des Mittels (a), ohne dass jedoch durch zu viel Wasser eine nennenswerte Oligomerisierung bzw. Polymerisierung initiiert würde.

Im Rahmen einer weiteren Ausführungform ist ein erfindungsgemäßes Verfahren dadurch gekennzeichnet, dass das Mittel (a) in Schritt (1) auf handtuchtrockenem oder trockenem keratinischen Material, ganz besonders bevorzugt auf handtuchtrockenem keratinischen Material, angewendet wird.

Im Rahmen einer weiteren Ausführungform ist ein erfindungsgemäßes Verfahren dadurch gekennzeichnet, dass das Mittel (a) in Schritt (1) auf handtuchtrockenen oder trockenen menschlichen Haaren, ganz besonders bevorzugt auf handtuchtrockenen menschlichen Haaren, angewendet wird.

Im Anschluss an das Auftragen auf das keratinische Material bzw. die Haare kann das Mittel (a) noch einmassiert werden. Das Einmassieren kann beispielsweise durch mechanisches Reiben oder Rubbeln der Haare mit der behandschuhten Hand erfolgen.

### Mittel (a)

Das Mittel (a) ist ein anwendungsbereites Mittel. Es ist dadurch gekennzeichnet, dass es - bezogen auf das Gesamtgewicht des Mittels (a) - weniger als 10 Gew.-% Wasser (a1) enthält, und mindestens eine organische Siliciumverbindung aus der Gruppe der Silane mit einem, zwei oder drei Siliciumatomen (a2) enthält und mindestens ein farbgebende Verbindung (a3) enthält.

Das Mittel (a) kann flüssig, gelartig oder cremeförmig sein. So kann das Mittel (a) beispielsweise in Form einer Creme, einer Emulsion, eines Gel oder auch einer Tensid-haltige schäumenden Lösung vorliegen, wobei diese jeweils weniger als 10 Gew.-% Wasser enthält.

### Wassergehalt (a1) im Mittel (a)

Bezogen auf das Gesamtgewicht des Mittels (a) enthält dieses weniger als 10 Gew.-% Wasser. Hierdurch wird gewährleistet, dass das Mittel (a) über den gesamten Anwendungszeitraum stabil bleibt und eine vorzeitige, unerwünschte Oligomerisierung bzw. Polymerizierung der Silane in ausreichendem Maße vermieden werden kann. Auch wenn die Stabilität des Mittels bereits bei einem Wassergehalt von bis zu 10 Gew.-% sichergestellt werden kann, so hat es sich zur weiteren Optimierung der Stabilität und der Farbintensitäten als bevorzugt herausgestellt, den Wassergehalt des Mittels (a) auf einen Wert unterhalb von 10 Gew.-% einzustellen. So wurden besonders gute Ergebnisse erhalten, wenn der Wassergehalt im Mittel (a) - bezogen auf das gesamtgewicht des Mittels (a) - bei 0 bis 7,5 Gew.-%, bevorzugt von 0,0 bis 5,0 Gew.-%, weiter bevorzugt von 0,0 bis 4,0 Gew.-% und ganz besonders bevorzugt 0 bis 2,5 Gew.-% lag.

Im Rahmen einer weiteren ganz besonders bevorzugten Ausführungsform ist ein erfindungsgemäßes Verfahren dadurch gekennzeichnet, dass das Mittel (a) - bezogen auf das Gesamtgewicht des Mittels (a) - von 0 bis 7,5 Gew.-%, bevorzugt von 0,0 bis 5,0 Gew.-%, weiter bevorzugt von 0,0 bis 4,0 Gew.-% und ganz besonders bevorzugt 0 bis 2,5 Gew.-% Wasser (a1) enthält.

Der Bereich von 0 bis 2,5 Gew.-% Wasser bedeutet, dass das Mittel möglichst kein Wasser enthält oder aber das die Wassermenge, die gegebenenfalls durch weitere im Mittel (a) enthaltene Inhaltsstoffe in das Mittel (a) eingeschleppt wird, einen Gehalt von 2,5 Gew.-% nicht überschreitet.

### Organische Siliciumverbindungen aus der Gruppe der Silane (a2) im Mittel (a)

Als zweiten erfindungswesentlichen Inhaltsstoff (a2) enthält das Mittel (a) mindestens eine organische Siliciumverbindung aus der Gruppe der Silane mit einem, zwei oder drei Siliciumatomen.

Besonders bevorzugt enthält das Mittel (a) mindestens eine organische Siliciumverbindung (a1), die aus Silanen mit einem, zwei oder drei Siliciumatomen ausgewählt ist, wobei die organische Siliciumverbindung eine oder mehrere Hydroxylgruppen und/oder hydrolysierbare Gruppen pro Molekül umfasst.

Diese im Mittel (a) enthaltenen organischen Siliciumverbindungen (a1) bzw. organischen Silane sind reaktive Verbindungen.

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist. Die erfindungsgemäßen organische Siliciumverbindungen sind Verbindungen, die eine bis drei Siliciumatome enthalten. Besonders bevorzugt enthalten die organische Siliciumverbindungen ein oder zwei Siliciumatome.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die Wasserstoff-Atome ganz oder teilweise durch organische Gruppen wie beispielsweise (substituierte) Alkylgruppen und/oder Alkoxygruppen ersetzt. In den organischen Silanen kann auch ein Teil der Wasserstoffatome durch Hydroxygruppen ersetzt sein.

Im Rahmen einer besonders bevorzugten Ausführungsform ist ein Verfahren gekennzeichnet durch die Anwendung eines Mittels (a) auf dem keratinischem Material, wobei das Mittel (a) mindestens eine organische Siliciumverbindung (a1) enthält, die aus Silanen mit einem, zwei oder drei Siliciumatomen ausgewählt ist, wobei die organische Siliciumverbindung außerdem eine oder mehrere Hydroxylgruppen oder hydrolysierbare Gruppen pro Molekül umfasst.

Im Rahmen einer ganz besonders bevorzugten Ausführungsform ist ein Verfahren gekennzeichnet durch die Anwendung eines Mittels (a) auf dem keratinischem Material, wobei das Mittel (a) mindestens eine organische Siliciumverbindung (a1) enthält, die aus Silanen mit einem, zwei oder drei Siliciumatomen ausgewählt ist, wobei die organische Siliciumverbindung außerdem eine oder mehrere basische chemische Funktionen und eine oder mehrere Hydroxylgruppen oder hydrolysierbare Gruppen pro Molekül umfasst.

Bei dieser basischen Gruppe oder basischen chemischen Funktion kann es sich beispielsweise um eine Aminogruppe, eine Alkylaminogruppe, eine Dialkylaminogruppe oder um eine Trialkylaminogruppe handeln, die bevorzugt über einen Linker mit einem Siliciumatom verbunden ist. Bevorzugt handelt es sich bei der basischen Gruppe um eine Aminogruppe, eine C₁-C₆-Alkylaminogruppe oder um eine Di(C₁-C₆)alkylaminogruppe.

Bei der oder den hydrolysierbaren Gruppen handelt es sich bevorzugt um eine C₁-C₆-Alkoxygruppe, insbesondere um eine Ethoxygruppe oder um eine Methoxygruppe. Es ist bevorzugt, wenn die hydrolysierbare Gruppe direkt an das Siliciumatom gebunden vorliegt. Handelt es sich beispielsweise bei der hydrolysierbaren Gruppe um eine Ethoxygruppe, so enthält die organische Siliciumverbindung bevorzugt eine Struktureinheit R'R"R‴Si-O-CH2-CH3. Die Reste R', R" und R‴ stellen hierbei die drei übrigen freien Valenzen des Siliciumatoms dar.

Ein ganz besonders bevorzugtes Verfahren ist dadurch gekennzeichnet, dass das Mittel (a) mindestens eine organische Siliciumverbindung enthält, die aus Silanen mit einem, zwei oder drei Siliciumatomen ausgewählt ist, wobei die organische Siliciumverbindung bevorzugt eine oder mehrere basische chemische Funktionen und eine oder mehrere Hydroxylgruppen oder hydrolysierbare Gruppen pro Molekül umfasst.

Ganz besonders gute Ergebnisse konnten erhalten werden, wenn das Mittel (a) mindestens eine organische Siliciumverbindung (a1) der Formel (I) und/oder (II) enthält.

Die Verbindungen der Formeln (I) und (II) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

In einer weiteren ganz besonders bevorzugten Ausführungsform ist das Verfahren dadurch gekennzeichnet, dass das Mittel (a) mindestens eine organische Siliciumverbindung (a2) der Formel (I) und/oder (II) enthält

R₁R₂N-L-Si(OR₃)ₐ(R₄)_{b} (I),

wobei
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃, R₄ unabhängig voneinander für eine C₁-C₆-Alkylgruppe stehen,
- a, für eine ganze Zahl von 1 bis 3 steht, und
- b für die ganze Zahl 3 - a steht, und
   wobei in der organischen Siliciumverbindung der Formel (II)

   (R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R5, R5', R5", R6, R6' und R6" unabhängig voneinander für eine C₁-C₆-Alkylgruppe stehen,
- A, A', A", A‴ und A"" unabhängig voneinander für eine lineare oder verzweigte, zweiwertige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkylgruppe oder eine Gruppierung der Formel (III) stehen
- (A"")-Si(R₆")_{d}"(OR₅")_{c}" (III),

   - c, für eine ganze Zahl von 1 bis 3 steht,
   - d für die ganze Zahl 3 - c steht,
   - c' für eine ganze Zahl von 1 bis 3 steht,
   - d' für die ganze Zahl 3 - c' steht,
   - c" für eine ganze Zahl von 1 bis 3 steht,
   - d" für die ganze Zahl 3 - c" steht,
   - e für 0 oder 1 steht,
   - f für 0 oder 1 steht,
   - g für 0 oder 1 steht,
   - h für 0 oder 1 steht,
mit der Maßgabe, dass mindestens einer der Reste aus e, f, g und h von 0 verschieden ist.

Die Substituenten R₁, R₂, R₃, R₄, R₅, R₅', R₅", R₆, R₆', R₆", R₇, R₈, L, A, A', A", A‴ und A"" in den Verbindungen der Formel (I) und (II) sind nachstehend beispielhaft erläutert:
Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Amino-C₁-C₆-alkyl-gruppe sind die Aminomethylgruppe, die 2-Aminoethylgruppe, die 3-Aminopropylgruppe. Die 2-Aminoethylgruppe ist besonders bevorzugt. Beispiele für eine lineare zweiwertige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweiwertige Alkylengruppen auch verzweigt sein. Beispiele für verzweigte, zweiwertige C₃-C₂₀-Alkylengruppen sind (-CH₂-CH(CH₃)-) und (-CH₂-CH(CH₃)-CH₂-).

In den organischen Siliciumverbindungen der Formel (I)

R₁R₂N-L-Si(OR₃)ₐ(R₄)_{b} (I),

stehen die Reste R₁ und R₂ unabhängig voneinander für ein Wasserstoffatom oder eine C₁-C₆-Alkylgruppe. Ganz besonders bevorzugt stehen die Reste R₁ und R₂ beide für ein Wasserstoffatom.

Im Mittelteil der organischen Siliciumverbindung befindet sich die Struktureinheit oder der Linker -L-der für eine lineare oder verzweigte, zweiwertige C₁-C₂₀-Alkylengruppe steht.

Eine zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung L zwei Bindungen eingehen kann. Eine Bindung erfolgt von der Aminogruppe R1R2N zum Linker L, und die zweite Bindung besteht zwischen dem Linker L und dem Siliciumatom.

Bevorzugt steht -L- für eine lineare, zweiwertige (d.h. divalente) C₁-C₂₀-Alkylengruppe. Weiter bevorzugt steht -L- für eine lineare zweiwertige C₁-C₆-Alkylengruppe. Besonders bevorzugt steht -L-für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt steht L für eine Propylengruppe (-CH₂-CH₂-CH₂-).

Die lineare Propylengruppe (-CH₂-CH₂-CH₂-) kann alternativ auch als Propan-1,3-diylgruppe bezeichnet werden.

Die organischen Siliciumverbindungen der Formel (I)

R₁R₂N-L-Si(OR₃)ₐ(R₄)_{b} (I),

tragen jeweils ein einem Ende die Silicium-haltige Gruppierung -Si(OR₃)ₐ(R₄)_{b}.

In der endständigen Struktureinheit -Si(OR₃)ₐ(R₄)_{b} steht der Rest R₃ für ein Wasserstoffatom oder eine C₁-C₆-Alkylgruppe, und der Rest R₄ steht für eine C₁-C₆-Alkylgruppe. Besonders bevorzugt stehen R₃ und R₄ unabhängig voneinander für eine Methylgruppe oder eine Ethylgruppe.

Hierbei steht a für eine ganze Zahl von 1 bis 3, und b steht für die ganze Zahl 3 - a. Wenn a für die Zahl 3 steht, dann ist b gleich 0. Wenn a für die Zahl 2 steht, dann ist b gleich 1. Wenn a für die Zahl 1 steht, dann ist b gleich 2.

Besonders widerstandsfähige Filme konnten erzeugt werden, wenn das Mittel (a) mindestens eine organische Siliciumverbindung (a2) der Formel (I) enthält, in welcher die Reste R₃, R₄ unabhängig voneinander für eine Methylgruppe oder für eine Ethylgruppe stehen.

Bei Einsatz des Verfahrens zum Färben von menschlichen Haaren konnten dann Färbungen mit den besten Waschechtheiten erhalten werden, wenn das Mittel (a) mindestens eine organische Siliciumverbindung (a2) der Formel (I) enthält, in welcher die Reste R₃, R₄ unabhängig voneinander für eine Methylgruppe oder für eine Ethylgruppe stehen.

Weiterhin konnten Färbungen mit den besten Waschechtheiten erhalten werden, wenn das Mittel (a) mindestens eine organische Siliciumverbindung der Formel (I) enthält, in welcher der Rest a für die Zahl 3 steht. In diesem Fall steht der Rest b für die Zahl 0.

In einer weiteren bevorzugten Ausführungsform ist das in dem Verfahren eingesetzte Mittel (a) dadurch gekennzeichnet, dass es mindestens eine organische Siliciumverbindung (a2) der Formel (I) enthält, wobei
- R₃, R₄ unabhängig voneinander für eine Methylgruppe oder für eine Ethylgruppe stehen und
- a für die Zahl 3 steht und
- b für die Zahl 0 steht.

In einer weiteren bevorzugten Ausführungsform ist ein erfindungsgemäßes Verfahren dadurch gekennzeichnet, dass das Mittel (a) mindestens eine organische Siliciumverbindung (a2) der Formel (I) enthält,

R₁R₂N-L-Si(OR₃)ₐ(R₄)_{b} (I),

wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht,
- R₃ für ein Wasserstoffatom, eine Ethylgruppe oder eine Methylgruppe steht,
- R₄ für eine Methylgruppe oder für eine Ethylgruppe steht,
- a für die Zahl 3 steht und
- b für die Zahl 0 steht.

Zur Lösung der erfindungsgemäßen Aufgabenstellung besonders gut geeignete organische Siliciumverbindungen der Formel (I) sind

### - (3-Aminopropyl)triethoxysilan

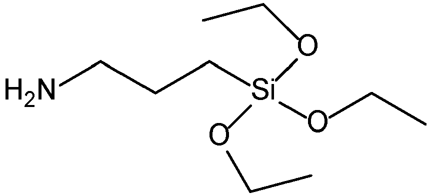

### - (3-Aminopropyl)trimethoxysilan

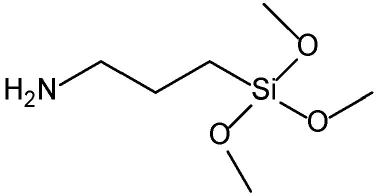

### - 1-(3-Aminopropyl)silantriol

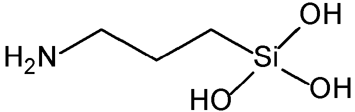

### - (2-Aminoethyl)triethoxysilan

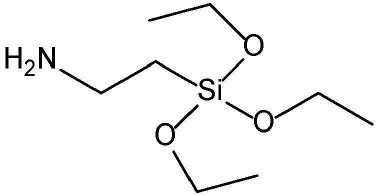

### - (2-Aminoethyl)trimethoxysilan

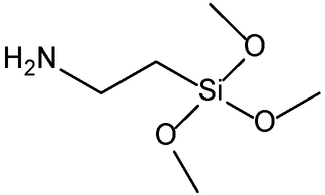

### 1-(2-Aminoethyl)silantriol

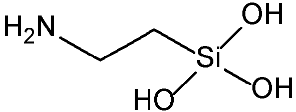

### - (3-Dimethylaminopropyl)triethoxysilan

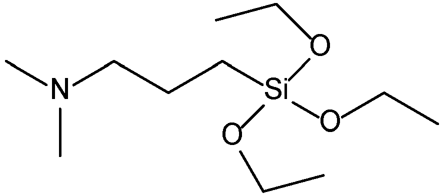

### - (3-Dimethylaminopropyl)trimethoxysilan

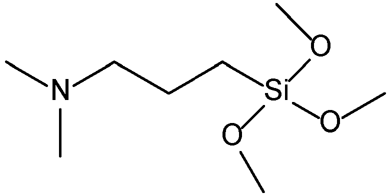

### 1-(3-Dimethylaminopropyl)silantriol

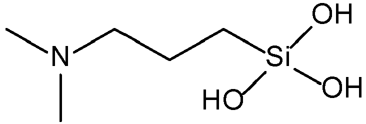

### - (2-Dimethylaminoethyl)triethoxysilan.

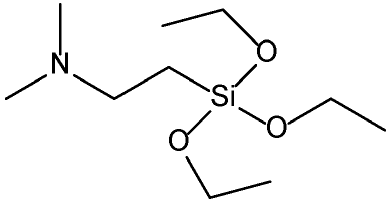

### - (2-Dimethylaminoethyl)trimethoxysilan und

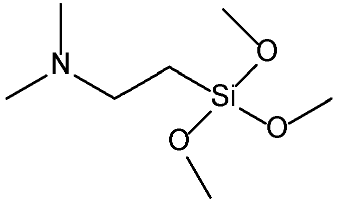

### 1-(2-Dimethylaminoethyl)silantriol

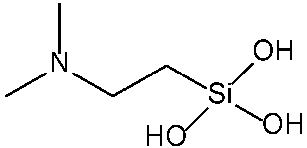

In einer weiteren bevorzugten Ausführungsform ist ein Verfahren dadurch gekennzeichnet, dass das Mittel (a) mindestens eine organische Siliciumverbindung (a2) der Formel (I) enthält, die ausgewählt ist aus der Gruppe aus
- (3-Aminopropyl)triethoxysilan
- (3-Aminopropyl)trimethoxysilan
- 1-(3-Aminopropyl)silantriol
- (2-Aminoethyl)triethoxysilan
- (2-Aminoethyl)trimethoxysilan
- 1-(2-Aminoethyl)silantriol
- (3-Dimethylaminopropyl)triethoxysilan
- (3-Dimethylaminopropyl)trimethoxysilan
- 1-(3-Dimethylaminopropyl)silantriol
- (2-Dimethylaminoethyl)triethoxysilan.
- (2-Dimethylaminoethyl)trimethoxysilan und/oder
- 1-(2-Dimethylaminoethyl)silantriol.

Die vorgenannten organischen Siliciumverbindungen der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erwerblich.

Im Rahmen einer weiteren Ausführungsform enthält das Mittel mindestens eine organische Siliciumverbindung (a2) der Formel (II)

(R₅O)_{c}(R₆)_{d}Si-(A)ₑ-[NR₇-(A')]_{f}-[O-(A")_{g}-[NR₈-(A‴)]ₕ-Si(R₆')_{d'}(OR₅')_{c'} (II).

Die siliciumorganischen Verbindungen der Formel (II) tragen jeweils an ihren beiden Enden die Silicium-haltigen Gruppierungen (R₅O)_{c}(R₆)_{d}Si- und -Si(R₆')_{d'}(OR₅')_{c'}.

Im Mittelteil des Moleküls der Formel (II) befinden sich die Gruppierungen -(A)ₑ- und -[NR₇-(A')]_{f}- und -[O-(A")]_{g}- und -[NR₈-(A‴)]ₕ-. Hierbei kann jeder der Reste e, f, g und h unabhängig voneinander für die Zahl 0 oder 1 stehen, wobei die Maßgabe besteht, dass mindestens einer der Reste e, f, g und h von 0 verschieden ist. Mit anderen Worten enthält eine organischen Siliciumverbindung der Formel (II) mindestens eine Gruppierung aus der Gruppe aus -(A)- und -[NR₇-(A')]- und -[O-(A")]- und -[NR₈-(A‴)]-.

In den beiden endständigen Struktureinheiten (R₅O)_{c}(R₆)_{d}Si- und - Si(R₆')_{d'}(OR₅')_{c'} stehen die Reste R5, R5', R5" unabhängig voneinander für ein Wasserstoffatom oder für eine C₁-C₆-Alkylgruppe. Die Reste R6, R6' und R6" stehen unabhängig voneinander für eine C₁-C₆-Alkylgruppe.

Hierbei steht c für eine ganze Zahl von 1 bis 3, und d steht für die ganze Zahl 3 - c. Wenn c für die Zahl 3 steht, dann ist d gleich 0. Wenn c für die Zahl 2 steht, dann ist d gleich 1. Wenn c für die Zahl 1 steht, dann ist d gleich 2.

Analog steht c' für eine ganze Zahl von 1 bis 3, und d' steht für die ganze Zahl 3 - c'. Wenn c' für die Zahl 3 steht, dann ist d' gleich 0. Wenn c' für die Zahl 2 steht, dann ist d' gleich 1. Wenn c' für die Zahl 1 steht, dann ist d' gleich 2.

Filme mit der höchsten Stabilität bzw. Färbungen mit den besten Waschechtheiten konnten erhalten werden, wenn die Reste c und c' beide für die Zahl 3 stehen. In diesem Fall stehen d und d' beide für die Zahl 0.

In einer weiteren bevorzugten Ausführungsform ist ein Verfahren dadurch gekennzeichnet, dass das Mittel (a) mindestens eine organische Siliciumverbindung (a2) der Formel (II) enthält,

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- R5 und R5' unabhängig voneinander für eine Methylgruppe oder eine Ethylgruppe stehen,
- c und c' beide für die Zahl 3 stehen und
- d und d' beide für die Zahl 0 stehen.

Wenn c und c' beide für die Zahl 3 stehen und d und d' beide für die Zahl 0 stehen, entsprechen die erfindungsgemäßen organischen Siliciumverbindung der Formel (IIa)

(R₅O)₃Si-(A)ₑ-[NR₇-(A')]_{f}-[O-(A")_{g}-[NR₈-(A‴)]ₕ-Si(OR₅')₃ (IIa).

Die Reste e, f, g und h können unabhängig voneinander für die Zahl 0 oder 1 stehen, wobei mindestens ein Rest aus e, f, g und h von null verschieden ist. Durch die Kürzel e, f, g und h wird demnach definiert, welche der Gruppierungen -(A)ₑ- und -[NR₇-(A')]_{f}- und -[O-(A")]_{g}- und -[NR₈-(A‴)]ₕ- sich im Mittelteil der organischen Siliciumverbindung der Formel (II) befinden.

In diesem Zusammenhang hat sich die Anwesenheit bestimmter Gruppierungen als besonders vorteilhaft im Hinblick auf die Erhöhung der Waschechtheit erwiesen. Besonders gute Ergebnisse konnten erhalten werden, wenn mindestens zwei der Reste e, f, g und h für die Zahl 1 stehen. Ganz besonders bevorzugt stehen e und f beide für die Zahl 1. Weiterhin ganz besonders bevorzugt stehen g und h beide für die Zahl 0.

Wenn e und f beide für die Zahl 1 stehen und g und h beide für die Zahl 0 stehen, entsprechen die erfindungsgemäßen organischen Siliciumverbindung der Formel (IIb)

(R₅O)_{c}(R₆)_{d}Si-(A)-[NR₇-(A')]-Si(R₆')_{d'}(OR₅')_{c'} (IIb).

Die Reste A, A', A", A‴ und A"" stehen unabhängig voneinander für eine lineare oder verzweigte, zweiwertige C₁-C₂₀-Alkylengruppe. Bevorzugt stehen die Reste A, A', A", A‴ und A"" unabhängig voneinander für eine lineare, zweiwertige C₁-C₂₀-Alkylengruppe. Weiter bevorzugt stehen die Reste A, A', A", A‴ und A"" unabhängig voneinander für eine lineare zweiwertige C₁-C₆-Alkylengruppe. Besonders bevorzugt stehen die Reste A, A', A", A‴ und A"" unabhängig voneinander für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt stehen die Reste A, A', A", A‴ und A"" für eine Propylengruppe (-CH₂-CH₂-CH₂-).

Die zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung A, A', A", A‴ und A"" zwei Bindungen eingehen kann.

Die lineare Propylengruppe (-CH₂-CH₂-CH₂-) kann alternativ auch als Propan-1,3-diylgruppe bezeichnet werden.

Wenn der Rest f für die Zahl 1 steht, dann enthält die organische Siliciumverbindung der Formel (II) eine strukturelle Gruppierung -[NR₇-(A')]-.

Wenn der Rest h für die Zahl 1 steht, dann enthält die organische Siliciumverbindung der Formel (II) eine strukturelle Gruppierung -[NR₈-(A‴)]-.

Hierbei stehen die Reste R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkylgruppe oder eine Gruppierung der Formel (III)
- (A"")-Si(R₆")_{d}"(OR₅")_{c}" (III).

Ganz besonders bevorzugt stehen die Reste R7 und R8 unabhängig voneinander für ein Wasserstoffatom, eine Methylgruppe, eine 2-Hydroxyethylgruppe, eine 2-Alkenylgruppe, eine 2-Aminoethylgruppe oder für eine Gruppierung der Formel (III).

Wenn der Rest f für die Zahl 1 steht und der Rest h für die Zahl 0 steht, enthält die organische Siliciumverbindung die Gruppierung [NR₇-(A')], aber nicht die Gruppierung -[NR₈-(A‴)]. Steht nun der Rest R7 für eine Gruppierung der Formel (III), so enthält das Mittel (a) eine organische Siliciumverbindung mit 3 reaktiven Silan-Gruppen.

In einer weiteren bevorzugten Ausführungsform ist ein Verfahren dadurch gekennzeichnet, dass das Mittel (a) mindestens eine organische Siliciumverbindung (a2) der Formel (II) enthält,

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- e und f beide für die Zahl 1 stehen,
- g und h beide für die Zahl 0 stehen,
- A und A' unabhängig voneinander für eine lineare, zweiwertige C₁-C₆-Alkylengruppe stehen und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

In einer weiteren bevorzugten Ausführungsform ist ein Verfahren dadurch gekennzeichnet, dass das Mittel (a) mindestens eine organische Siliciumverbindung (a2) der Formel (II) enthält, wobei
- e und f beide für die Zahl 1 stehen,
- g und h beide für die Zahl 0 stehen,
- A und A' unabhängig voneinander für eine Methylengruppe (-CH₂-), eine Ethylengruppe (-CH₂-CH₂-) oder eine Propylengruppe (-CH₂-CH₂-CH₂) stehen, und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

Zur Lösung der erfindungsgemäßen Aufgabenstellung gut geeignete organische Siliciumverbindungen der Formel (II) sind

### - 3-(Trimethoxysilyl)-N-[3-(trimethoxysilyl)propyl]-1-propanamin

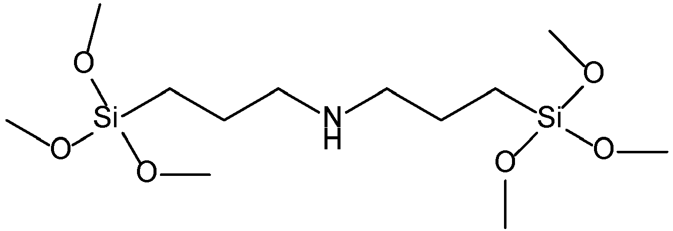

### - 3-(Triethoxysilyl)-N-[3-(triethoxysilyl)propyl]-1-propanamin

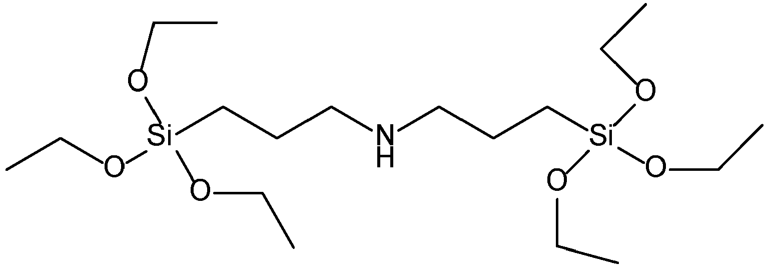

### - N-Methyl-3-(trimethoxysilyl)-N-[3-(trimethoxysilyl)propyl]-1-propanamin

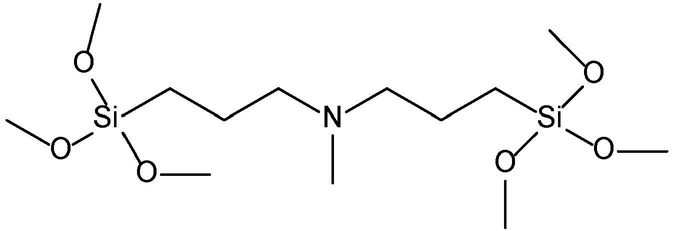

### - N-Methyl-3-(triethoxysilyl)-N-[3-(triethoxysilyl)propyl]-1-propanamin

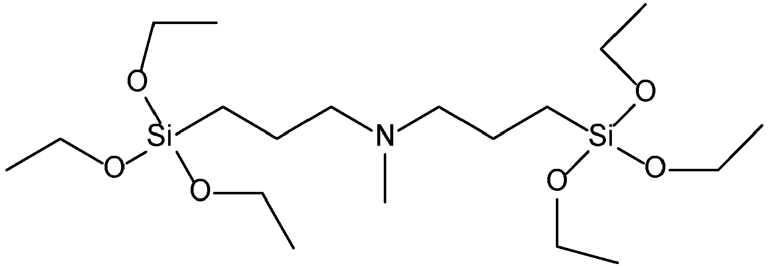

### - 2-[Bis[3-(trimethoxysilyl)propyl]amino]-ethanol

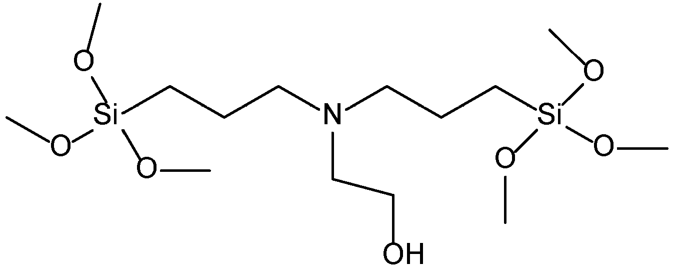

### - 2-[Bis[3-(triethoxysilyl)propyl]amino]-ethanol

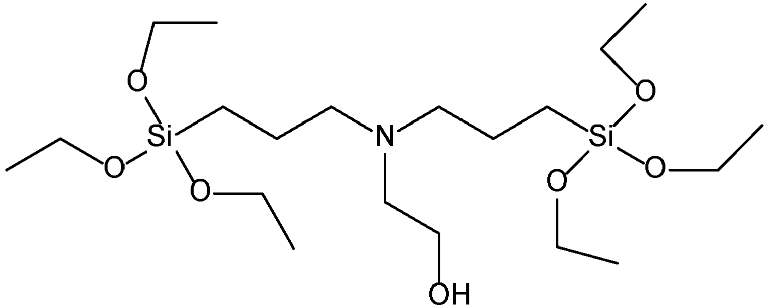

### - 3-(Trimethoxysilyl)-N,N-bis[3-(trimethoxysilyl)propyl]-1-propanamin

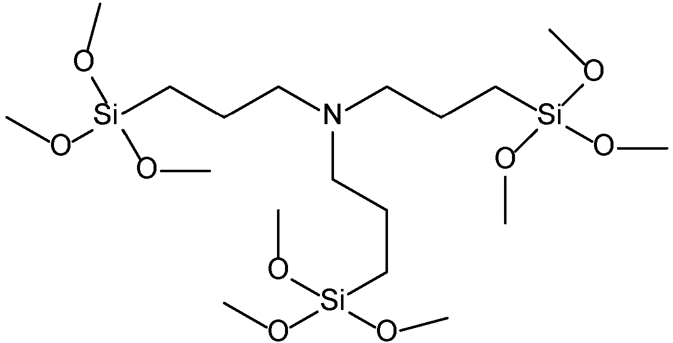

### - 3-(Triethoxysilyl)-N,N-bis[3-(triethoxysilyl)propyl]-1-propanamin

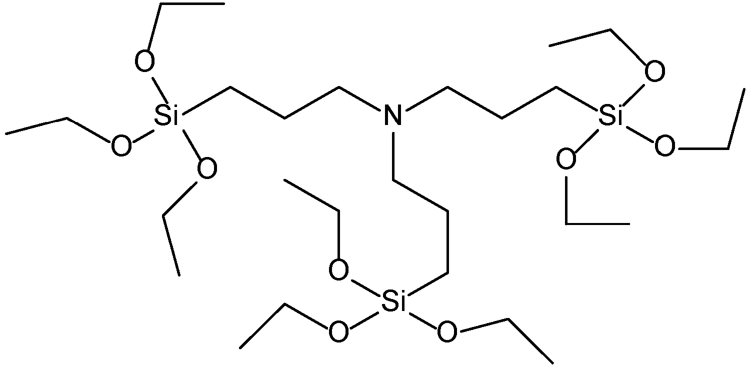

### - N1,N1-Bis[3-(trimethoxysilyl)propyl]-1,2-ethanediamin,

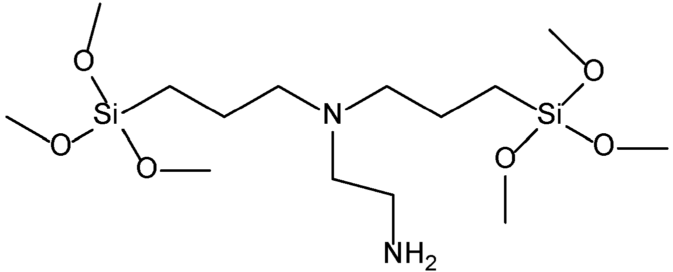

### - N1,N1-Bis[3-(triethoxysilyl)propyl]-1,2-ethanediamin,

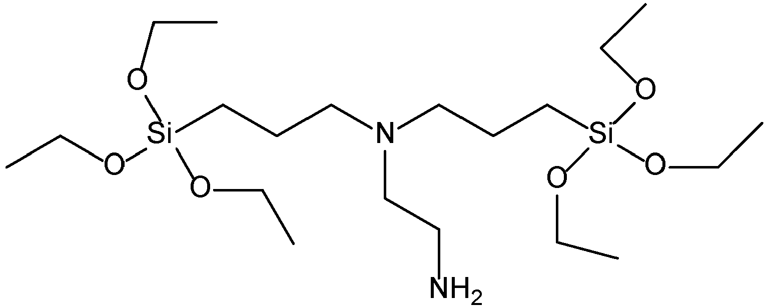

### - N,N-Bis[3-(trimethoxysilyl)propyl]-2-propen-1-amin

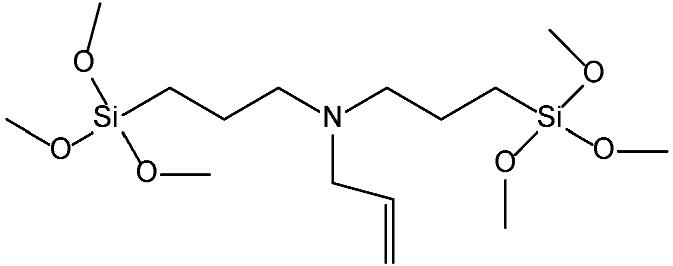

### - N,N-Bis[3-(triethoxysilyl)propyl]-2-propen-1-amin

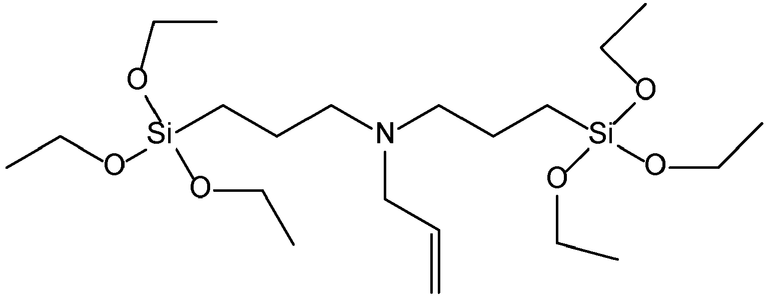

Die vorgenannten organische Siliciumverbindung der Formel (II) sind kommerziell erhältlich. Bis(trimethoxysilylpropyl)amine mit der CAS-Nummer 82985-35-1 kann beispielsweise von Sigma-Aldrich käuflich erworben werden.

Bis[3-(triethoxysilyl)propyl]amine mit der CAS-Nummer 13497-18-2 kann zum Beispiel von Sigma-Aldrich käuflich erworben werden.

N-Methyl-3-(trimethoxysilyl)-N-[3-(trimethoxysilyl)propyl]- 1-propanamin wird alternativ auch als Bis(3-trimethoxysilylpropyl)-N-methylamin bezeichnet und kann bei Sigma-Aldrich oder Fluorochem kommerziell erworben werden.

3-(Triethoxysilyl)-N,N-bis[3-(triethoxysilyl)propyl]-1-propanamin mit der CAS-Nummer 18784-74-2 kann beispielsweise von Fluorochem oder Sigma-Aldrich käuflich erworben werden.

In einer weiteren bevorzugten Ausführungsform ist ein Verfahren dadurch gekennzeichnet, dass das Mittel (a) mindestens eine organische Siliciumverbindung (a2) enthält, die ausgewählt ist aus der Gruppe aus
- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1-propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1,N1-Bis[3-(trimethoxysilyl)propyl]-1,2-Ethanediamin,
- N1,N1-Bis[3-(triethoxysilyl)propyl]-1,2-Ethanediamin,
- N,N-Bis[3-(trimethoxysilyl)propyl]-2-Propen-1-amin und/oder
- N,N-Bis[3-(triethoxysilyl)propyl]-2-Propen-1-amin.

In weiteren Färbeversuchen, hat es sich ebenfalls als ganz besonders vorteilhaft herausgestellt, wenn das in dem Verfahren auf dem keratinischen Material angewendete Mittel (a) mindestens eine organische Siliciumverbindung (a2) der Formel (IV) enthält

R₉Si(OR₁₀)ₖ(R₁₁)ₘ (IV)

.

Die Verbindungen der Formel (IV) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

Das bzw. die organischen Siliciumverbindungen der Formel (IV) können auch als Silane vom Typ der Alkylalkoxysilane oder der Alkylhydroxysilane bezeichnet werden,

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform ist ein erfindungsgemäßes Verfahren dadurch gekennzeichnet, dass das Mittel (a) mindestens eine organische Siliciumverbindung (a2) der Formel (IV) enthält.

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren ganz besonders bevorzugten Ausführungsform ist ein erfindungsgemäßes Verfahren dadurch gekennzeichnet, dass das Mittel (a) zusätzlich zu der oder den organischen Siliciumverbindungen der Formel (I) mindestens eine weitere organische Siliciumverbindung der Formel (IV) enthält

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform ist ein Verfahren dadurch gekennzeichnet, dass das Mittel (a) zusätzlich zu der oder den organischen Siliciumverbindungen der Formel (II) mindestens eine weitere organische Siliciumverbindung der Formel (IV) enthält

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform ist ein Verfahren dadurch gekennzeichnet, dass das Mittel (a) zusätzlich zu der oder den organischen Siliciumverbindungen der Formel (I) und/oder (II) mindestens eine weitere organische Siliciumverbindung der Formel (IV) enthält

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In den organischen Siliciumverbindungen der Formel (IV) steht der Rest R₉ für eine C₁-C₁₈-Alkylgruppe. Diese C₁-C₁₈-Alkylgruppe ist gesättigt und kann linear oder verzweigt sein. Bevorzugt steht R₉ für eine lineare C₁-C₁₈-Alkylgruppe. Bevorzugt steht R₉ für eine Methylgruppe, eine Ethylgruppe, eine n-Propylgruppe, eine n-Butylgruppe, eine n-Pentylgruppe, eine n-Hexylgruppe, eine n-Octylgruppe, eine n-Dodecylgruppe oder eine n-Octadecylgruppe. Besonders bevorzugt steht R₉ für eine Methylgruppe, eine Ethylgruppe, eine n-Hexylgruppe oder eine n-Octylgruppe.

In den organischen Siliciumverbindungen der Form (IV) steht der Rest R₁₀ für ein Wasserstoffatom oder eine C₁-C₆-Alkylgruppe. Besonders bevorzugt steht R₁₀ für eine Methylgruppe oder für eine Ethylgruppe.

In den organischen Siliciumverbindungen der Form (IV) steht der Rest R₁₁ für eine C₁-C₆-Alkylgruppe. Besonders bevorzugt steht R₁₁ für eine Methylgruppe oder für eine Ethylgruppe.

Weiterhin steht k für eine ganze Zahl von 1 bis 3, und m steht für die ganze Zahl 3 - k. Wenn k für die Zahl 3 steht, dann ist m gleich 0. Wenn k für die Zahl 2 steht, dann ist m gleich 1. Wenn k für die Zahl 1 steht, dann ist m gleich 2.

Besonders stabile Filme, d.h. Färbungen mit besonders guten Waschechtheiten konnten erhalten werden, wenn im Verfahren ein Mittel (a) eingesetzt wurde, welches mindestens eine organische Siliciumverbindung (a1) der Formel (IV) enthält, in welcher der Rest k für die Zahl 3 steht. In diesem Fall steht der Rest m für die Zahl 0.

Zur Lösung der erfindungsgemäßen Aufgabenstellung besonders gut geeignete organische Siliciumverbindungen der Formel (IV) sind

### - Methyltrimethoxysilan

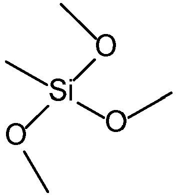

### - Methyltriethoxysilan

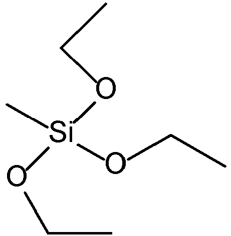

### - Ethyltrimethoxysilan

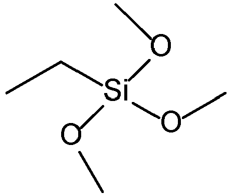

### - Ethyltriethoxysilan

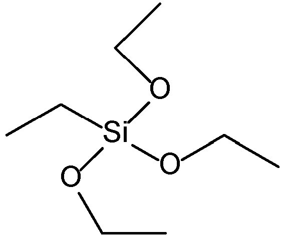

### - n-Hexyltrimethoxysilan

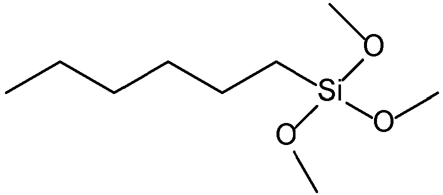

### - n-Hexyltriethoxysilan

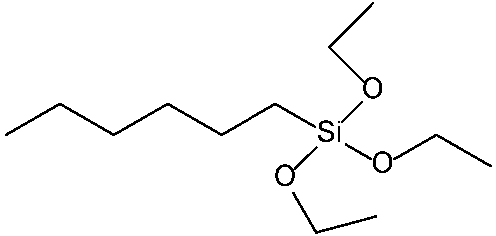

### - n-Octyltrimethoxysilan

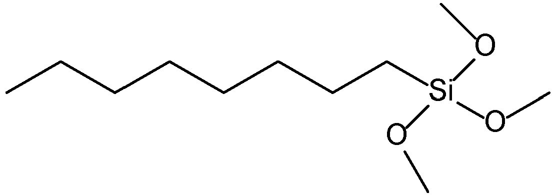

### - n-Octyltriethoxysilan

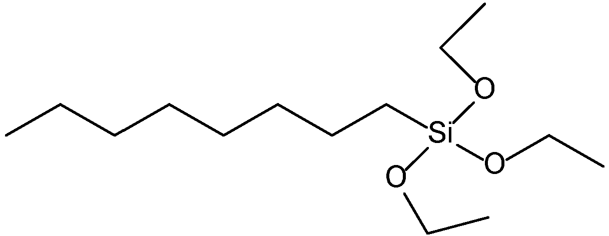

### - n-Dodecyltrimethoxysilan und/oder

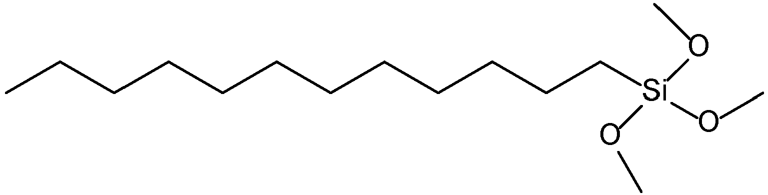

### - n-Dodecyltriethoxysilan.

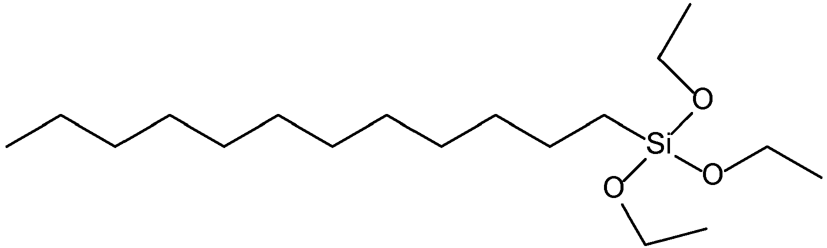

### n-Octadecyltrimethoxysilan und/oder n-Octadecyltriethoxysilan.

In einer weiteren bevorzugten Ausführungsform ist ein erfindungsgemäßes Verfahren dadurch gekennzeichnet, dass das Mittel (a) mindestens eine organische Siliciumverbindung (a2) der Formel (IV) enthält, die ausgewählt ist aus der Gruppe aus
- Methyltrimethoxysilan
- Methyltriethoxysilan
- Ethyltrimethoxysilan
- Ethyltriethoxysilan
- Propyltrimethoxysilan
- Propyltriethoxysilan
- Hexyltrimethoxysilan
- Hexyltriethoxysilan
- Octyltrimethoxysilan
- Octyltriethoxysilan
- Dodecyltrimethoxysilan,
- Dodecyltriethoxysilan,
- Octadecyltrimethoxysilan und
- Octadecyltriethoxysilan.

Bei den zuvor beschriebenen organischen Siliciumverbindungen handelt es sich um reaktive Verbindungen. In diesem Zusammenhang hat es sich als bevorzugt herausgestellt, wenn das Mittel (a) - bezogen auf das Gesamtgewicht des Mittels (a) - eine oder mehrere organische Siliciumverbindungen (a2) in einer Gesamtmenge von 0,1 bis 20 Gew.-%, bevorzugt 1 bis 15 Gew.-% und besonders bevorzugt 2 bis 8 Gew.-% enthält.

In einer weiteren bevorzugten Ausführungsform ist ein Verfahren dadurch gekennzeichnet, dass das Mittel (a) - bezogen auf das Gesamtgewicht des Mittels (a) - eine oder mehrere organische Siliciumverbindungen (a2) in einer Gesamtmenge von 0,1 bis 20 Gew.-%, bevorzugt 1 bis 15 Gew.-% und besonders bevorzugt 2 bis 8 Gew.-% enthält.

Zur Erzielung von besonders guten Färbeergebnissen ist es insbesondere von Vorteil, die organische Siliciumverbindungen der Formel (I) und/oder (II) in bestimmten Mengenbereichen im Mittel (a) einzusetzen. Besonders bevorzugt enthält das Mittel (a) - bezogen auf das Gesamtgewicht des Mittels (a) - ein oder mehrere organische Siliciumverbindungen (a2) der Formel (I) und/oder (II) in einer Gesamtmenge von 0,1 bis 10 Gew.-%, bevorzugt 0,5 bis 5 Gew.-% und besonders bevorzugt 0,5 bis 3 Gew.-%.

In einer weiteren bevorzugten Ausführungsform ist ein Verfahren dadurch gekennzeichnet, dass das Mittel (a) - bezogen auf das Gesamtgewicht des Mittels (a) - eine oder mehrere organische Siliciumverbindungen (a2) der Formel (I) und/oder (II) in einer Gesamtmenge von 0,1 bis 10 Gew.-%, bevorzugt 0,5 bis 5 Gew.-% und besonders bevorzugt 0,5 bis 3 Gew.-% enthält.

Weiterhin hat es sich als ganz besonders bevorzugt herausgestellt, wenn auch das oder die organische Siliciumverbindungen der Formel (IV) in bestimmten Mengenbereichen im Mittel (a) enthalten sind. Besonders bevorzugt enthält das Mittel (a) - bezogen auf das Gesamtgewicht des Mittels (a) - eine oder mehrere organische Siliciumverbindungen (a2) der Formel (IV) in einer Gesamtmenge von 0,1 bis 20 Gew.-%, bevorzugt 2 bis 15 Gew.-% und besonders bevorzugt 4 bis 9 Gew.-%.

In einer weiteren bevorzugten Ausführungsform ist ein Verfahren dadurch gekennzeichnet, dass das Mittel (a) - bezogen auf das Gesamtgewicht des Mittels (a) - eine oder mehrere organische Siliciumverbindungen (a2) der Formel (IV) in einer Gesamtmenge von 0,1 bis 20 Gew.-%, bevorzugt 2 bis 15 Gew.-% und besonders bevorzugt 3,2 bis 10 Gew.-% enthält.

Im Zuge der zu dieser Erfindung führenden Arbeiten hat sich herausgestellt, dass auf dem keratinischen Material auch dann besonders stabile und gleichmäßige Filme erhalten werden konnten, wenn das Mittel (a) zwei strukturell voneinander verschiedene organische Siliciumverbindungen enthält.

In einer weiteren bevorzugten Ausführungsform ist ein erfindungsgemäßes Verfahren daher dadurch gekennzeichnet, dass das Mittel (a) mindestens zwei strukturell voneinander verschiedene organische Siliciumverbindungen (a2) enthält.

In einer bevorzugten Ausführungsform ist ein Verfahren dadurch gekennzeichnet, dass auf dem keratinischen Material ein Mittel (a) angewendet wird, welches mindestens eine organische Siliciumverbindungen der Formel (I) und mindestens eine organische Siliciumverbindungen der Formel (IV) enthält.

In einer explizit ganz besonders bevorzugten Ausführungsform ist ein Verfahren dadurch gekennzeichnet, dass auf dem keratinischen Material ein Mittel (a) angewendet wird, welches mindestens eine organische Siliciumverbindungen der Formel (I) enthält, welche aus der Gruppe aus (3-Aminopropyl)triethoxysilan und (3-Aminopropyl)trimethoxysilan ausgewählt ist, und zusätzlich mindestens eine organische Siliciumverbindungen der Formel (IV) enthält, welche aus der Gruppe bestehend aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan und Hexyltriethoxysilan ausgewählt ist.

In einer weiteren bevorzugten Ausführungsform ist ein Verfahren dadurch gekennzeichnet, dass das Mittel (a) - bezogen auf das Gesamtgewicht des Mittels (a) - enthält:
- 0,5 bis 5 Gew.-% mindestens einer ersten organischen Siliciumverbindung (a2) die ausgewählt ist aus der Gruppe aus (3-Aminopropyl)trimethoxysilan, (3-Aminopropyl)triethoxysilan, (2-Aminoethyl)trimethoxysilan, (2-Aminoethyl)triethoxysilan, (3-Dimethylaminopropyl)trimethoxysilan, (3-Dimethylaminopropyl)triethoxysilan (2-Dimethylaminoethyl)trimethoxysilan und (2-Dimethylaminoethyl)triethoxysilan, und
- 3,2 bis 10 Gew.-% mindestens einer zweiten organischen Siliciumverbindung (a2) die ausgewählt ist aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und Octadecyltriethoxysilan.

Im Rahmen dieser Ausführungsform enthält das Mittel (a) eine oder mehrere organischen Siliciumverbindungen einer ersten Gruppe in einer Gesamtmenge von 0,5 bis 3 Gew.-%. Die organischen Siliciumverbindungen dieser ersten Gruppe sind ausgewählt aus der Gruppe aus (3-Aminopropyl)trimethoxysilan, (3-Aminopropyl)triethoxysilan, (2-Aminoethyl)trimethoxysilan, (2-Aminoethyl)triethoxysilan, (3-Dimethylaminopropyl)trimethoxysilan, (3-Dimethylaminopropyl)-triethoxysilan (2-Dimethylaminoethyl)trimethoxysilan und/oder (2-Dimethylaminoethyl)-triethoxysilan.

Im Rahmen dieser Ausführungsform enthält das Mittel (a) eine oder mehrere organischen Siliciumverbindungen einer zweiten Gruppe in einer Gesamtmenge von 3,2 bis 10 Gew.-%. Die organischen Siliciumverbindungen dieser zweiten Gruppe sind ausgewählt aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und Octadecyltriethoxysilan.

### Oligomere und/oder Kondensationsprodukte der siliciumorganischen Verbindungen (a2)

Bei den zuvor beschriebenen organischen Siliciumverbindungen aus der Gruppe der Silane mit einem, zwei oder drei Siliciumatomen (a2) führt bereits der Zusatz geringer Wassermengen zur Hydrolyse bzw. zur Oligimerisierung und/oder Polymerisierung. Das Ausmaß der Oligomerisierung bzw. Polymerisierung ist hierbei abhängig von der Menge an Wasser, die mit dem oder den Silanen (a2) in Kontakt kommt. Ziel des erfindungsgemäßen Verfahrens ist, dass die Ausbildung des gefärbten Films, d.h. die von den Silanen (a2) ausgehende finale Polymerisierung, erst in Schritt (2) des Verfahrens erfolgt, wenn das Mittel (a) sich bereits auf dem Keratinmaterial befindet. Dennoch kann bedingt durch die hohe Reaktivität der Silane (a2) bereits vor Anwendung des Mittels (a) eine Oligomisierung oder Vorkondensation stattgefunden haben, und die Silane (a2) können im Mittel (a) bereits oligomerisiert bzw. in geringen Anteilen auch bereits polymerisiert sein.

Aus diesem Grund können sowohl die Silane mit einem, zwei oder drei Siliciumatomen (a2) als auch deren Oligomere und/oder Kondensationsprodukte im Mittel (a) enthalten sein. Erfindungs-gemäß sind daher vom Begriff "Silane mit einem, zwei oder drei Siliciumatomen (a2)" auch deren Hydrolyseprodukte, Oligomere und/oder deren Kondensationsprodukte umfasst.

Bei den entsprechenden Hydrolyse, Oligomeren und/oder Kondensationsprodukten handelt es sich beispielsweise um die folgenden Verbindungen. Hierbei stellen die Kondensationsprodukte maximal oligomere Verbindungen, jedoch keine Polymere dar.

Hydrolyse von C₁-C₆-Alkoxy-Silan der Formel (S-I) mit Wasser (Reaktionsschema am Beispiel von 3-Aminopropyltriethoxysilan):

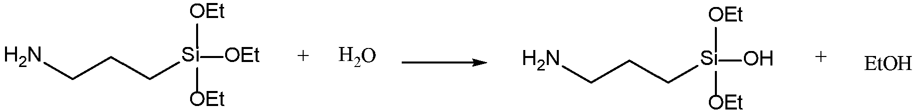

In Abhängigkeit von der eingesetzten Menge an Wasser kann die Hydrolyse-Reaktion auch mehrfach pro eingesetztem C₁-C₆-Alkoxy-Silan stattfinden: 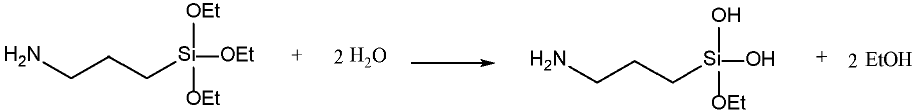 bzw.

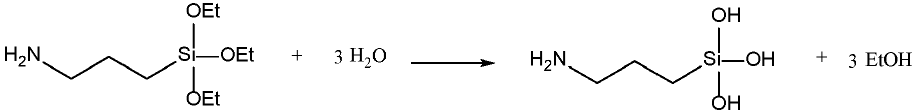

Hydrolyse von C₁-C₆-Alkoxy-Silan der Formel (S-IV) mit Wasser (Reaktionsschema am Beispiel von Methyltrimethoxysilan):

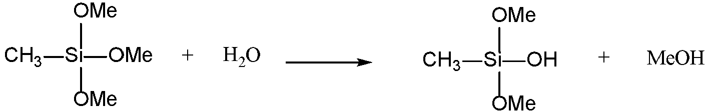

In Abhängigkeit von der eingesetzten Menge an Wasser kann die Hydrolyse-Reaktion auch mehrfach pro eingesetztem C₁-C₆-Alkoxy-Silan stattfinden: 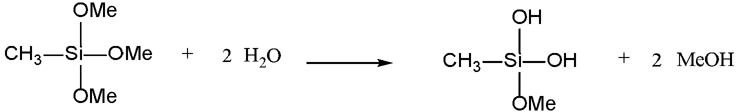 bzw.

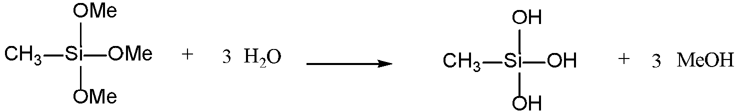

Mögliche Kondensationsreaktionen sind beispielsweise (gezeigt anhand des Gemisches (3-Aminopropyl)triethoxysilan und Methyltrimethoxysilan): 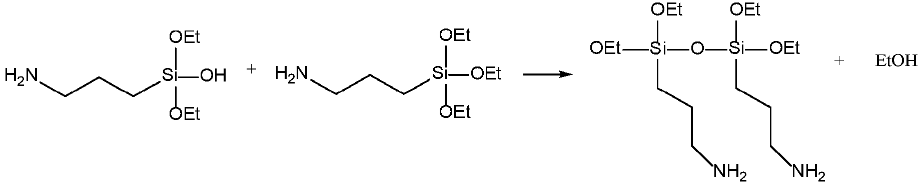 und/oder 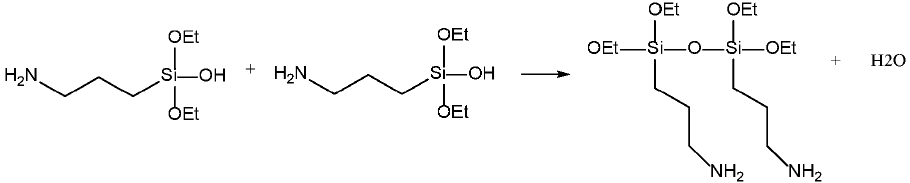 und/oder 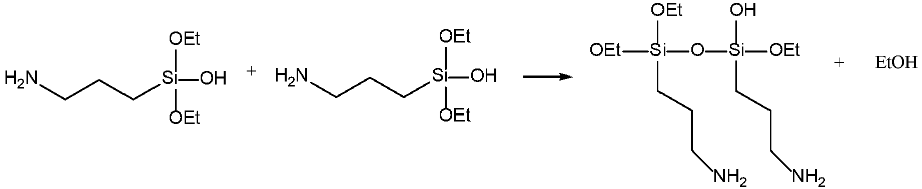 und/oder 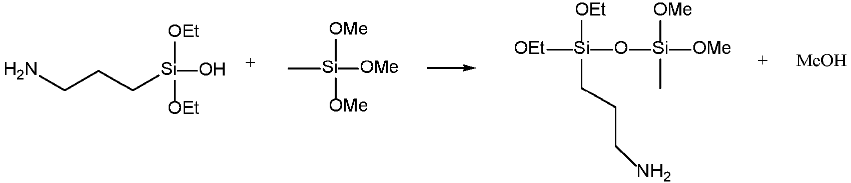 und/oder 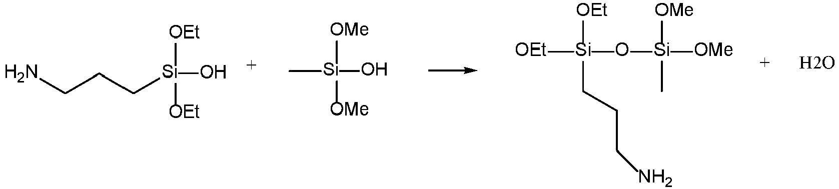 und/oder 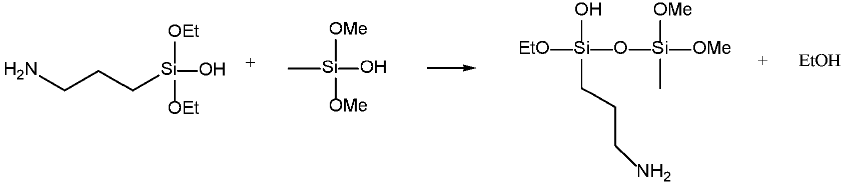 und/oder

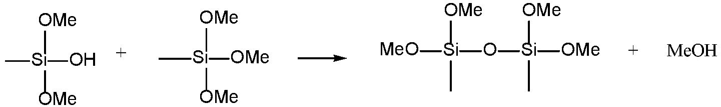

In den obigen beispielhaften Reaktionsschemata ist jeweils die Kondensation zu einem Dimer gezeigt, jedoch sind auch weitergehende Kondensationen zu Oligomeren mit mehreren Silan-Atomen möglich und auch bevorzugt.

Unter einem Kondensationsprodukt wird ein Produkt verstanden, dass durch Reaktion von mindestens zwei organischen Siliciumverbindungen mit jeweils mindestens einer Hydroxylgruppen oder hydrolysierbaren Gruppen pro Molekül unter Abspaltung von Wasser und/oder unter Abspaltung von einem Alkanol entsteht. Die Kondensationsprodukte können beispielsweise Dimere, aber auch Trimere oder Oligomere sein, wobei die Kondensationsprodukte mit den Monomeren im Gleichgewicht stehen. Abhängig von der eingesetzten oder in der Hydrolyse verbrauchten Wassermenge verschiebt sich das Gleichgewicht von monomerer organischen Siliciumverbindungen zu Kondensationsprodukt.

Ganz besonders gute Ergebnisse konnten erhalten werden, wenn in dem Verfahren organischen Siliciumverbindungen der Formel (I) und/oder (II) und/oder (IV) eingesetzt wurden. Da wie bereits zuvor beschrieben bereits bei Spuren von Feuchtigkeit eine Hydrolyse/Kondensation einsetzt, sind damit auch die Hydrolyse- und/oder Kondensationsprodukte der organischen Siliciumverbindungen (I) und/oder (II) und/oder (IV) von dieser Ausführungsform mit umfasst.

### farbgebende Verbindungen (a3) im Mittel (a)

Als dritten erfindungswesentlichen Bestandteil enthält das Mittel (a) mindestens eine farbgebende Verbindung. Bei der Ausbildung des durch die Polymerisierung der Silane (a2) entstehenden Films werden die farbgebende(n) Verbindung(en) in diesen eingelagert und auf diese Weise an der Keratinoberlfäche fixiert. Bei solchen farbgebenden Verbindungen handelt es sich ganz besonders bevorzugt um Pigmente und/oder direktziehende Farbstoffe, ganz besonders bevorzugt um Pigmente.

Im Rahmen einer weiteren besonders bevorzugten Ausführungsform ist ein erfindungsgemäßes Verfahren dadurch gekennzeichnet, dass das Mittel (a) mindestens eine farbgebende Verbindung (a3) aus der Gruppe aus Pigmenten und/oder direktziehenden Farbstoffen enthält.

Im Rahmen einer weiteren besonders bevorzugten Ausführungsform ist ein erfindungsgemäßes Verfahren dadurch gekennzeichnet, dass das Mittel (a) mindestens ein Pigment (a3) enthält.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Pigmente können anorganischen und/oder organischen Ursprungs sein.

In einer bevorzugten Ausführungsform ist ein Verfahren dadurch gekennzeichnet, dass das Mittel (a) mindestens eine farbgebende Verbindung (a3) aus der Gruppe der anorganischen und/oder organischen Pigmente enthält.

Bevorzugte Pigmente sind ausgewählt aus synthetischen oder natürlichen anorganischen Pigmenten. Anorganische Pigmente natürlichen Ursprungs können beispielsweise aus Kreide, Ocker, Umbra, Grünerde, gebranntem Terra di Siena oder Graphit hergestellt werden. Weiterhin können als anorganische Pigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Pigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Ebenfalls besonders bevorzugte Pigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Entsprechend ist ein bevorzugtes Verfahren dadurch gekennzeichnet, dass das Mittel (a) mindestens ein anorganisches Pigment (a3) enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

Ein bevorzugt geeignetes Pigment auf Basis von synthetischem Glimmer ist beispielsweise Timiron^{®} SynWhite Satin von Merck.

In einer weiteren bevorzugten Ausführungsform ist das Verfahren dadurch gekennzeichnet, dass das Mittel (a) und/oder das Mittel (b) mindestens eine farbgebende Verbindung aus der Gruppe der Pigmente enthält, die ausgewählt ist aus Pigmenten auf Basis von natürlichem oder synthetischem Glimmer, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Weitere geeignete Pigmente basieren auf Metalloxid-beschichteten plättchenförmigen Borosilikaten. Diese sind beispielsweise mit Zinnoxid, Eisenoxid(en), Siliciumdioxid und/oder Titandioxid beschichtet. Solche Borosilikat-basierten Pigmente sind beispielsweise unter der Bezeichnung MIRAGE von Eckart oder Reflecks von BASF SE erhältlich.

Beispiele für besonders geeignete Pigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} oder SynCrystal von der Firma Eckart Cosmetic Colors, Flamenco^{®}, Cellini^{®}, Cloisonné^{®}, Duocrome^{®}, Gemtone^{®}, Timica^{®}, MultiReflections, Chione von der Firma BASF SE und Sunshine^{®} von der Firma Sunstar erhältlich.

Ganz besonders bevorzugte Pigmente mit der Handelsbezeichnung Colorona^{®} sind beispielsweise:
Colorona Copper, Merck, MICA, CI 77491 (IRON OXIDES)
Colorona Copper Fine, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491 (Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491 (Iron oxides)
Colorona Blackstar Gold, Merck, MICA, CI 77499 (IRON OXIDES)
Colorona^{®} SynCopper, Merck, Synthetic Fluorphlogopite (and) Iron Oxides
Colorona^{®} SynBronze, Merck, Synthetic Fluorphlogopite (and) Iron Oxides

Weiterhin besonders bevorzugte Pigmente mit der Handelsbezeichnung Xirona^{®} sind beispielsweise:
Xirona^{®} Golden Sky, Merck, Silica, CI 77891 (Titanium Dioxide), Tin Oxide
Xirona^{®} Caribbean Blue, Merck, Mica, CI 77891 (Titanium Dioxide), Silica, Tin Oxide
Xirona^{®} Kiwi Rose, Merck, Silica, CI 77891 (Titanium Dioxide), Tin Oxide
Xirona^{®} Magic Mauve, Merck, Silica, CI 77891 (Titanium Dioxide), Tin Oxide
Xirona^{®} Le Rouge, Merck, Iron Oxides (and) Silica

Zudem sind besonders bevorzugte Pigmente mit der Handelsbezeichnung Unipure^{®} beispielsweise:
Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Ebenfalls besonders bevorzugte Pigmente mit der Handelsbezeichnung Flamenco^{®} sind beispielsweise:
Flamenco^{®} Summit Turquoise T30D, BASF, Titanium Dioxide (and) Mica
Flamenco^{®} Super Violet 530Z, BASF, Mica (and) Titanium Dioxide

Im Rahmen einer weiteren Ausführungsform kann das in dem Verfahren eingesetzte Mittel (a) und/oder Mittel (b) auch ein oder mehrere farbgebende Verbindungen aus der Gruppe der organischen Pigmente enthalten.

Bei den organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform erfindungsgemäßes Verfahren dadurch gekennzeichnet, dass das Mittel (a) mindestens ein organisches Pigment (a3) enthält, das bevorzugt ausgewählt ist aus der Gruppe aus Carmin, Chinacridon, Phthalocyanin, Sorgho, blauen Pigmenten mit den Color Index Nummern CI 42090, CI 69800, CI 69825, CI 73000, CI 74100, CI 74160, gelben Pigmenten mit den Color Index Nummern CI 11680, CI 11710, CI 15985, CI 19140, CI 20040, CI 21100, CI 21108, CI 47000, CI 47005, grünen Pigmenten mit den Color Index Nummern CI 61565, CI 61570, CI 74260, orangen Pigmenten mit den Color Index Nummern CI 11725, CI 15510, CI 45370, CI 71105, roten Pigmenten mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Zur Färbung des Keratinmaterials können auch Pigmente mit einer bestimmten Formgebung eingesetzt worden sein. Beispielsweise kann ein Pigment auf Basis eines lamellaren und/oder eines lentikularen Substratplättchens eingesetzt werden. Weiterhin ist auch die Färbung auf Basis eines Substratplättchens möglich, welches ein Vakuum metallisiertes Pigment umfasst.

Im Rahmen einer weiteren Ausführungsform ist ein erfindungsgemäßes Verfahren dadurch gekennzeichnet, dass das Mittel (a) ein oder mehrere farbgebende Verbindungen (a3) aus der Gruppe der Pigmente auf Basis eines lamellaren Substratplättchens, der Pigmente auf Basis eines lentikularen Substratplättchens und der Vakuum metallisierten Pigmente enhält.

Die Substratplättchen dieses Typs weisen eine durchschnittliche Dicke von höchstens 50 nm, vorzugsweise weniger als 30 nm, besonders bevorzugt höchstens 25 nm, beispielsweise höchstens 20 nm auf. Die durchschnittliche Dicke der Substratplättchen beträgt mindestens 1 nm, vorzugsweise mindestens 2,5 nm, besonders bevorzugt mindestens 5 nm, beispielsweise mindestens 10 nm. Bevorzugte Bereiche für die Dicke der Substratplättchen sind 2,5 bis 50 nm, 5 bis 50 nm, 10 bis 50 nm; 2,5 bis 30 nm, 5 bis 30 nm, 10 bis 30 nm; 2,5 bis 25 nm, 5 bis 25 nm, 10 bis 25 nm, 2,5 bis 20 nm, 5 bis 20 nm und 10 bis 20 nm. Vorzugsweise weist jedes Substratplättchen eine möglichst einheitliche Dicke auf.

Durch die geringe Dicke der Substratplättchen weist das Pigment ein besonders hohes Deckvermögen auf.

Die Substratplättchen sind bevorzugt monolithisch aufgebaut. Monolithisch bedeutet in diesem Zusammenhang aus einer einzigen abgeschlossenen Einheit ohne Brüche, Schichtungen oder Einschlüsse bestehend, wobei jedoch innerhalb der Substratplättchen Gefügewechsel auftreten können. Die Substratplättchen sind vorzugsweise homogen aufgebaut, d.h. dass innerhalb der Plättchen kein Konzentrationsgradient auftritt. Insbesondere sind die Substratplättchen nicht schichtartig aufgebaut und weisen keine darin verteilten Teilchen oder Partikel auf.

Die Größe des Substratplättchens kann auf den jeweiligen Anwendungszweck, insbesondere dem gewünschten Effekt auf dem keratinischen Material, abgestimmt werden. In der Regel haben die Substratplättchen einen mittleren größten Durchmesser von etwa 2 bis 200 µm, insbesondere etwa 5 bis 100 µm.

In einer bevorzugten Ausführungsform beträgt der Formfaktor (Aspect Ratio), ausgedrückt durch das Verhältnis der mittleren Größe zur durchschnittlichen Dicke, mindestens 80, vorzugsweise mindestens 200, mehr bevorzugt mindestens 500, besonders bevorzugt mehr als 750, beträgt. Dabei wird als mittlere Größe der unbeschichteten Substratplättchen der d50-Wert der unbeschichteten Substratplättchen verstanden. Der d50-Wert wurde, soweit nicht anders angegeben, mit einem Gerät des Typs Sympatec Helos mit Quixel-Nassdispergierung bestimmt. Dabei wurde zur Probenvorbereitung die zu untersuchende Probe für eine Dauer von 3 Minuten in Isopropanol vordispergiert.

Die Substratplättchen können aus jedem Material, das in Plättchenform gebracht werden kann, aufgebaut sein.

Sie können natürlichen Ursprungs, aber auch synthetisch hergestellt sein. Materialien, aus denen die Substratplättchen aufgebaut sein können, sind beispielsweise Metalle und Metalllegierungen, Metalloxide, vorzugsweise Aluminiumoxid, anorganische Verbindungen und Mineralien wie Glimmer und (Halb)Edelsteine, sowie Kunststoffe. Vorzugsweise sind die Substratplättchen aus Metall(legierung)en aufgebaut.

Als Metall kommt jedes für metallische Glanzpigmente geeignete Metall in Betracht. Derartige Metalle sind unter anderem Eisen und Stahl, sowie alle luft- und wasserbeständigen (Halb)metalle wie beispielsweise Platin, Zink, Chrom, Molybdän und Silicium, sowie deren Legierungen wie Aluminiumbronzen und Messing. Bevorzugte Metalle sind Aluminium, Kupfer, Silber und Gold. Bevorzugte Substratplättchen sind Aluminiumplättchen und Messingplättchen, wobei Substratplättchen aus Aluminium besonders bevorzugt sind.

Lamellare Substratplättchen zeichnen sich durch einen unregelmäßig strukturierten Rand aus und werden aufgrund ihres Erscheinungsbildes auch als "cornflakes" bezeichnet.

Aufgrund ihrer unregelmäßigen Struktur erzeugen Pigmente auf der Basis von lamellaren Substratplättchen einen hohen Anteil an Streulicht. Außerdem decken die Pigmente auf der Basis von lamellaren Substratplättchen die vorhandene Farbe eines keratinischen Materials nicht vollständig ab und es können beispielsweise Effekte analog zu einer natürlichen Ergrauung erzielt werden.

Lentikulare (= linsenförmige) Substratplättchen weisen einen im Wesentlichen regelmäßigen runden Rand auf und werden aufgrund ihres Erscheinungsbildes auch als "silverdollars" bezeichnet. Aufgrund ihrer regelmäßigen Struktur überwiegt bei Pigmenten auf Basis von lentikularen Substratplättchen der Anteil des reflektierten Lichts.

Vakuum metallisierte Pigmente *(vacuum metallized pigments,* VMP) können beispielsweise durch das Freisetzen von Metallen, Metalllegierungen oder Metalloxiden von entsprechend beschichteten Folien gewonnen werden. Sie zeichnen sich durch eine besonders geringe Dicke der Substratplättchen im Bereich von 5 bis 50 nm und durch eine besonders glatte Oberfläche mit erhöhter Reflektivität aus. Substratplättchen, welche ein im Vakuum metallisiertes Pigment umfassen, werden im Rahmen dieser Anmeldung auch als VMP-Substratplättchen bezeichnet. VMP-Substratplättchen aus Aluminium können beispielsweise durch Freisetzen von Aluminium von metallisierten Folien gewonnen werden.

Die Substratplättchen aus Metall oder Metalllegierung können passiviert sein, beispielsweise durch Eloxieren (Oxidschicht) oder Chromatieren.

Unbeschichtete lamellare, lentikulare und/oder VPM-Substratplättchen, insbesondere solche aus Metall oder Metalllegierung, reflektieren das einfallende Licht in hohem Maße und erzeugen einen Hell-Dunkel-Flop. Diese haben sich zum Einsatz im Mittel (a) als besonders bevorzugt erwiesen.

Geeignete Pigmente auf Basis eines lamellaren Substratplättchens umfassen beispielsweise die Pigmente der Reihe VISIONAIRE von Eckart.

Pigmente auf Basis eines lentikularen Substratplättchens sind beispielsweise unter der Bezeichnung Alegrace^{®} Gorgeous von der Firma Schlenk Metallic Pigments GmbH erhältlich.

Pigmente auf Basis eines Substratplättchens, welches ein Vakuum metallisiertes Pigment umfasst, sind beispielsweise unter der Bezeichnung Alegrace^{®} Marvelous oder Alegrace^{®} Aurous von der Firma Schlenk Metallic Pigments GmbH erhältlich.

Im Rahmen einer weiteren Ausführungsform ist ein erfindungsgemäßes Verfahren dadurch gekennzeichnet, dass das Mittel (a) - bezogen auf das Gesamtgewicht des Mittels (a) - ein oder mehrere Pigmente in einer Gesamtmengen von 0,001 bis 20 Gew.-%, insbesondere von 0,05 bis 5 Gew.-%, enthält.

Als farbgebende Verbindung(en) können die in dem Verfahren angewendeten Mittel (a) auch einen oder mehrere direktziehende Farbstoffe enthalten. Bei direktziehenden Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1 g/L.

Direktziehende Farbstoffe können in anionische, kationische und nichtionische direktziehende Farbstoffe unterteilt werden.

In einer weiteren bevorzugten Ausführungsform ist das Verfahren dadurch gekennzeichnet, dass das Mittel (a) als farbgebende Verbindung (a3) mindestens einen anionischen, kationischen und/oder nichtionischen direktziehenden Farbstoff enthält.

Geeignete kationische direktziehende Farbstoffe sind beispielsweise Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 und Basic Red 76.

Als nichtionische direktziehende Farbstoffe können beispielsweise nichtionische Nitro- und Chinonfarbstoffe und neutrale Azofarbstoffe eingesetzt werden. Geeignete nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung (-SO₃H) besitzen. In Abhängigkeit vom pH-Wert liegen die protonierten Formen (-COOH, -SO₃H) der Carbonsäure- bzw. Sulfonsäuregruppierungen mit ihren deprotonierten Formen (-COO-, -SO₃⁻vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Die Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Als gut geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, lodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intensität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriumsalze von Mono- und Disulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatriumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 Gew.-%.

Acid Red 33 ist das Dinatriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1 ,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

Der oder die direktziehenden Farbstoffe, insbesondere die anionischen direktziehenden Farbstoffe, können je nach erwünschter Farbintensität in verschiedenen Mengen in dem Mittel (a) eingesetzt werden. Gute Ergebnisse konnten erhalten werden, wenn das Mittel (a) - bezogen auf das Gesamtgewicht des Mittels (a) -ein oder mehrere direktziehende Farbstoffe (a3) in einer Gesamtmenge von 0,01 bis 10 Gew.-%, bevorzugt von 0,1 bis 8 Gew.-%, weiter bevorzugt von 0,2 bis 6 Gew.-% und ganz besonders bevorzugt von 0,5 bis 4,5 Gew.-% enthält.

### kosmetischer Träger (a4) im Mittel (a)

Das Mittel (a) enthält die organischen Silane (a2) und die farbgebenden Verbindungen (a3) besonders bevorzugt in einem kosmetischen Träger. Da das Mittel (a) zudem wasserarm bzw. wasserfrei ist, handelt es sich bei diesem kosmetischen Träger nicht um Wasser. Als kosmetische Träger besonders gut geeignet sind zum Beispiel die Verbindungen aus der Gruppe aus Poly-C₁-C₆-Alkylenglycolen, 1,2-Propylenglycol, 1,3-Propylenglycol, 1,2-Butylenglycol, Dipropylen-glycol, Ethanol, Isopropanol, Diethylenglycolmonoethylether, Glycerin, Phenoxyethanol und Benzylalkohol. Poly-C₁-C₆-Alkylenglycole, insbesondere Polyethylenglycole, haben hierbei eine ganz besonders gute Eignung gezeigt.

Im Rahmen einer weiteren besonders bevorzugten Ausführungsform ist ein erfindungsgemäßes Verfahren dadurch gekennzeichnet, dass das Mittel (a) mindestens einen kosmetischen Träger (a4) aus der Gruppe aus Poly-C₁-C₆-Alkylenglycolen, 1,2-Propylenglycol, 1,3-Propylenglycol, 1,2-Butylenglycol, Dipropylenglycol, Ethanol, Isopropanol, Diethylenglycolmonoethylether, Glycerin, Phenoxyethanol und Benzylalkohol, ganz besonders bevorzugt aus Polyethylenglycolen, enthält.

1,2-Propylenglycol wird alternativ auch als 1,2-Propandiol bezeichnet wird und trägt die CAS-Nummern 57-55-6 [(RS)-1,2-Dihydroxypropan], 4254-14-2 [(R)-1,2-Dihydroxypropan] und 4254-153 [(S)-1,2-Dihydroxypropan]. Ethylenglycol wird alternativ auch als 1,2-Ethandiol bezeichnet und trägt die CAS-Nummer 107-21-1.Glycerin wird alternativ auch als 1,2,3-Propantriol bezeichnet und trägt die CAS-Nummer 56-81-5. Phenoxyethanol besitzt die Cas-Nummer 122-99-6.

Alle zuvor beschriebenen Lösungsmittel sind bei verschiedenen Chemikalien-Lieferanten wie beispielsweise Aldrich oder Fluka kommerziell erhältlich.

Bei einem ganz besonders gut geeigneten Lösungsittel handelt es sich um Alkylenglycole der Formel (AG) 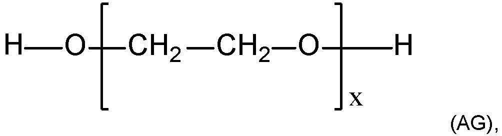 wobei
- x: für eine ganze Zahl von 1 bis 10000, bevorzugt für eine ganze Zahl von 2 bis 800, weiter bevorzugt für eine ganze Zahl von 3 bis 600, noch weiter bevorzugt für eine ganze Zahl von 3 bis 400 und ganz besonders bevorzugt für eine ganze Zahl von 4 bis 200 steht,

Im Rahmen einer weiteren ganz besonders bevorzugten Ausführungsform ist ein erfindungsgemäßes Verfahren daher dadurch gekennzeichnet, dass das Mittel (a) ein oder mehrere Alkylenglycole (a4) der Formel (AG) enthält, 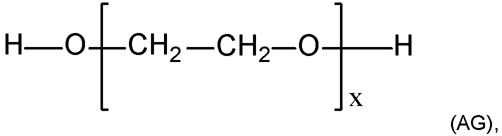 wobei
- x: für eine ganze Zahl von 1 bis 10000, bevorzugt für eine ganze Zahl von 2 bis 800, weiter bevorzugt für eine ganze Zahl von 3 bis 600, noch weiter bevorzugt für eine ganze Zahl von 3 bis 400 und ganz besonders bevorzugt für eine ganze Zahl von 4 bis 200 steht.

Bei den Alkylenglycolen der Formel (AG) handelt es sich um protische Substanzen mit mindestens einer Hydroxy-Gruppe, die aufgrund ihrer Wiederholungseinheit -CH2-CH2-O- , sofern x für einen Wert von mindestens 2 steht, auch als Polyalkylenglycole bzw. Polyethylenglycole bezeichnet werden können. In den Alkylenglycolen der Formel (AG) steht x für eine ganze Zahl von 1 bis 10000. Im Rahmen der zu dieser Erfindung führenden Arbeiten hat sich herausgestellt, dass diese Polyethylenglycole eine besonders gute Eignung zeigen, um zum einen die Echtheitseigenschaften der Färbemittel zu verbessern und zum anderen auch um die Viskosität der Mittel optimal einzustellen.

Abhängig von ihrer Kettenlänge sind Polyethylenglycole flüssige oder feste, wasserlösliche Polymere. Polyethylenglycole mit einer Molekülmasse zwischen 200 g/mol und 400 g/mol sind bei Raumtemperatur nichtflüchtige Flüssigkeiten. PEG 600 weist einen Schmelzbereich von 17 bis 22 °C und somit eine pastenartige Konsistenz auf. Bei Molekülmassen über 3000 g/mol sind die PEG feste Substanzen und werden als Schuppen oder Pulver in den Handel gebracht.

Vor allem der Einsatz von niedermolekularen Alkylenglycolen (bzw. Polyethylenglycolen) hat sich zur Lösung der erfindungsgemäßen Aufgabenstellung gut geeignet erwiesen. Bei niedermolekularen Alkylenglycolen (bzw. Polyethylenglycolen) im Sinne der vorliegenden Erfindung steht x für eine ganze Zahl von 1 bis 100, bevorzugt für eine ganze Zahl von 1 bis 80, weiter bevorzugt für eine ganze Zahl von 2 bis 60, noch weiter bevorzugt für eine ganze Zahl von 3 bis 40, noch weiter bevorzugt für eine ganze Zahl von 4 bis 20 und ganz besonders bevorzugt für eine ganze Zahl von 6 bis 15.

Im Rahmen einer weiteren ganz besonders bevorzugten Ausführungsform ist ein erfindungsgemäßes Mittel dadurch gekennzeichnet, dass es mindestens ein Alkylenglycol (a4) der Formel (AG-1) enthält, 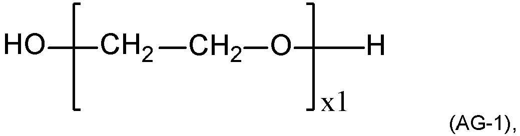 wobei
- x1: für eine ganze Zahl von 1 bis 100, bevorzugt für eine ganze Zahl von 1 bis 80, weiter bevorzugt für eine ganze Zahl von 2 bis 60, noch weiter bevorzugt für eine ganze Zahl von 3 bis 40, noch weiter bevorzugt für eine ganze Zahl von 4 bis 20 und ganz besonders bevorzugt für eine ganze Zahl von 6 bis 15 steht.

Ein ganz besonders bevorzugtes niedermolekulares Polyethylenglycol ist beispielsweise PEG-8. PEG-8 umfasst im Schnitt 8 Ethylenglycol-Einheiten (x1 = 8), besitzt ein mittleres Molgewicht von 400 g/mol und trägt die CAS-Nummer 25322-68-3. PEG-8 wird alternativ auch als PEG 400 bezeichnet und ist beispielsweise von der Firma APS kommerziell erhältlich.

Weitere gut geeignete niedermolekulare Polyethylenglycole sind beispielsweise PEG-6, PEG-7, PEG-9 und PEG-10.

Ein weiteres gut geeignetes Polyethylenglycol ist beispielsweise PEG-32. PEG-32 umfasst 32 Ethylenglycol-Einheiten (x1 = 32), besitzt ein mittleres Molgewicht von 1500 g/mol und trägt die CAS-Nummer 25322-68-3. PEG-32 wird alternativ auch als PEG 1500 bezeichnet und kann zum Beispiel von der Firma Clariant kommerziell erworben werden.

Weiterhin hat sich auch der Einsatz von hochmolekularen Polyethylenglycolen zur Lösung der erfindungsgemäßen Aufgabenstellung gut geeignet erwiesen.

Hochmolekulare Polyethylenglycole im Sinne der vorliegenden Erfindung können durch die Formel (AG-2) dargestellt werden, wobei die Indexzahl x2 für eine ganze Zahl von 101 bis 10000 steht

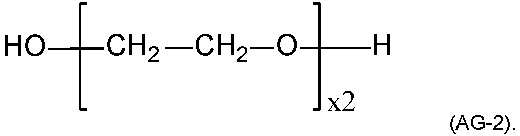

Bei ganz besonders gut geeigneten hochmolekularen Polyethylenglycolen steht x2 für eine ganze Zahl von 101 bis 1000, bevorzugt für eine ganze Zahl von 105 bis 800, weiter bevorzugt für eine ganze Zahl von 107 bis 600, noch weiter bevorzugt für eine ganze Zahl von 109 bis 400 und ganz besonders bevorzugt für eine ganze Zahl von 110 bis 200.

Im Rahmen einer weiteren ganz besonders bevorzugten Ausführungsform ist ein erfindungsgemäßes Mittel dadurch gekennzeichnet, dass es mindestens ein Alkylenglycol (a4) der Formel (AG-2) enthält, 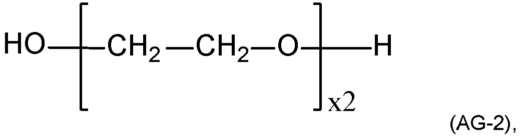 wobei
- x2: für eine ganze Zahl von 101 bis 1000, bevorzugt für eine ganze Zahl von 105 bis 800, weiter bevorzugt für eine ganze Zahl von 107 bis 600, noch weiter bevorzugt für eine ganze Zahl von 109 bis 400 und ganz besonders bevorzugt für eine ganze Zahl von 110 bis 200 steht.

Ein ganz besonders gut geeignetes hochmolekulares Polyethylenglycol ist beispielsweise PEG 6000, welches von der Firma National Starch (China) kommerziell erhalten werden kann. Das Molgewicht von PEG 6000 liegt bei 6000 bis 7500 g/mol, dies entspricht einem x2-Wert von 136 bis 171.

Ein weiteres gut geeignetes Polyethylenglycol ist PEG 12000, das zum Beispiel unter dem Handelsnamen Polyethylene Glycol 12000 S (oder PEG 12000 S) von der Firme CG Chemikalien kommerziell vertrieben wird. Das Molgewicht von PEG 12000 wird mit 10500 bis 15000 g/mol angegeben, entsprechend einem x2-Wert von 238 bis 341.

Ein weiteres gut geeignetes Polyethylenglycol ist auch PEG 20000, welches unter dem Handelsnamen Polyglycol 20000 P bzw. unter dem Alternativnamen PEG-350 von der Firma Clariant käuflich zu erwerblich ist. Für PEG 20000 wird ein Molgewicht von im Schnitt 20000 g/mol angegeben, dies entspricht einem x2-Wert von 454.

Bevorzugt enthält das Mittel (a) - bezogen auf das Gesamtgewicht des Mittels (a) - den oder die zuvor beschriebenen kosmetischen Träger (a4) in in einer Gesamtmenge von 10,0 bis 99,0 Gew.%, bevorzugt von 30,0 bis 99,0 Gew.-%, weiter bevorzugt von 50,0 bis 99,0 Gew.-% und ganz besonders bevorzugt von 70,0 bis 99,0 Gew.-%.

Im Rahmen einer weiteren ganz besonders bevorzugten Ausführungsform ist ein erfindungsgemäßes Verfahren dadurch gekennzeichnet, dass das Mittel (a) - bezogen auf das Gesamtgewicht des Mittels (a) - ein oder mehrere Bestandteile des kosmetischen Trägers (a4) in einer Gesamtmenge von 10,0 bis 99,0 Gew.-%, bevorzugt von 30,0 bis 99,0 Gew.-%, weiter bevorzugt von 50,0 bis 99,0 Gew.-% und ganz besonders bevorzugt von 70,0 bis 99,0 Gew.-% enthält.

Im Rahmen einer weiteren besonders bevorzugten Ausführungsform ist ein erfindungsgemäßes Verfahren dadurch gekennzeichnet, dass das Mittel (a) - bezogen auf das Gesamtgewicht des Mittels (a) - ein oder mehrere Bestandteile (a4) aus der Gruppe aus Poly-C₁-C₆-Alkylenglycolen, 1,2-Propylenglycol, 1,3-Propylenglycol, 1,2-Butylenglycol, Dipropylenglycol, Ethanol, Isopropanol, Diethylenglycolmonoethylether, Glycerin, Phenoxyethanol und Benzylalkohol in einer Gesamtmenge von 10,0 bis 99,0 Gew.-%, bevorzugt von 30,0 bis 99,0 Gew.-%, weiter bevorzugt von 50,0 bis 99,0 Gew.-% und ganz besonders bevorzugt von 70,0 bis 99,0 Gew.-% enthält.

Im Rahmen einer weiteren explizit ganz besonders bevorzugten Ausführungsform ist ein erfindungsgemäßes Verfahren dadurch gekennzeichnet, dass das Mittel (a) - bezogen auf das Gesamtgewicht des Mittels (a) - ein oder mehrere Alkylenglycole (a4) der Formel (AG) in einer Gesamtmenge von 10,0 bis 99,0 Gew.-%, bevorzugt von 30,0 bis 99,0 Gew.-%, weiter bevorzugt von 50,0 bis 99,0 Gew.-% und ganz besonders bevorzugt von 70,0 bis 99,0 Gew.-% enthält.

Hierbei versteht es sich, dass die Summe aus allen im Mitteln (a) enthaltenen Inhaltsstoffen aus den Gruppen (a1), (a2), (a3) und gegebenenfalls (a4) bei nicht mehr als 100 Gew.-% liegen kann. Sollen noch weitere, optionale Inhaltsstoffe im Mittel eingesetzt werden, so verringert sich die Gesamtsumme aus der vorgenannten Inhaltsstoffe in entsprechendem Ausmaß auf Werte von unter 100 Gew.-%.

### Schritt (2), Anwendung des Mittels (b) auf dem keratinischen Material

Im zweiten Schritt des erfindungsgemäßen Verfahrens wird das Mittel (b) auf das keratinische Material bzw. die Haare appliziert, die noch mit dem Mittel (a) beaufschlagt sind. Schritt (2) erfolgt nach Schritt (1), d.h. nachdem das Mittel (a) vollständig auf das Keratinmaterial aufgetragen wurde, wird das Mittel (b) appliziert. Das sukzessive Auftragen der beiden Mittel (a) und (b) erfolgt innerhalb eines Zeitraums von einer Stunde, bevorzugt innerhalb von 30 Minuten. Ganz besonders bevorzugt wird das Mittel (b) innerhalb von 10 Minuten, ganz besonders bevorzugt innerhalb von 5 Minuten nach Anwendung des Mittels (a) auf das Keratinmaterial appliziert. Ein Auftragen des Mittels (b) innerhalb von 10 Minuten bedeutet, dass mit dem Auftragen des Mittels (b) innerhalb eines Zeitraums von maximal 10 Minuten begonnen wird, nachdem das Auftragen des Mittels (a) auf das Keratinmaterial abgeschlossen ist.

Im Rahmen einer weiteren besonders bevorzugten Ausführungsform ist ein erfindungsgemäßes Verfahren gekennzeichnet durch die Anwendung des Mittels (b) innerhalb von 10 Minuten, bevorzugt innerhalb von 5 Minuten nach Anwendung des Mittels (a).

Durch das Auftragen des Mittels (b) wird an den Stellen des Keratinmaterials, die mit dem Mittel (a) beaufschlagt sind, eine Mischung der Mittel (a) und (b) hergestellt. Als wesentlichen Bestandteil enthält das Mittel (b) eine definierte Wassermenge (b1), die dazu führt, dass die nun auf dem Keratinmaterial befindlichen Silane (a2) die Oligomerisierungs- bzw. Polymerisations-Reaktion eingehen, die zur Ausbildung des gefärbten Films führt. Wesentlich für das erfindungsgemäße Verfahren ist damit die Ausbildung der Mischung aus (a) und (b) an all den Stellen des Keratinmaterials, die gefärbt werden sollen.

Durch das Vermischen der Mittel (a) und (b) werden die beiden Mittel miteinder emulgiert, so dass die Silane (a2) und die die Polymerisation initierende Wassermenge (b1) in direkten Kontakt miteinander treten. Das Vermischen kann beispielsweise manuell mit der behandschuhten Hand durch Einmassieren oder Einarbeiten der beiden Mittel in das Keratinmaterial erfolgen.

Im Rahmen einer weiteren besonders bevorzugten Ausführungsform ist ein erfindungsgemäßes Verfahren demnach gekennzeichnet durch die
(2) Anwendung eines Mittels (b) auf dem keratinischem Material, das noch mit dem Mittel (a) beaufschlagt ist, und Herstellung einer Mischung aus beiden Mitteln (a) und (b) auf dem Keratinmaterial durch manuelles Vermischen beider Mittel.

Das im Mittel (b) enthaltene Wasser initiiert die Polymerisation. Hierfür wird der Wassergehalt im Mittel (b) besonders bevorzugt auf bestimmte Werte eingestellt. In diesem Zusammenhang hat es sich als besonders vorteilhaft herausgestellt, wenn das Mittel (b) - bezogen auf das Gesamt-gewicht des Mittels (b) - 10 bis 100 Gew.-%, bevorzugt 30 bis 99,5 Gew.-%, weiter bevorzugt 50 bis 99 Gew.-% und ganz besonders bevorzugt 80 bis 99 Gew.-% Wasser enthält.

Im Rahmen einer weiteren besonders bevorzugten Ausführungsform ist ein erfindungsgemäßes Verfahren dadurch gekennzeichnet, dass das Mittel (b) - bezogen auf das Gesamtgewicht des Mittels (b) - 10 bis 100 Gew.-%, bevorzugt 30 bis 99,5 Gew.-%, weiter bevorzugt 50 bis 99 Gew.-% und ganz besonders bevorzugt 80 bis 99 Gew.-% Wasser enthält.

### Verdicker im Mittel (b)

Um eine ausreichend hohe Vermischbarkeit der Mittel (a) und (b) zu gewährleisten und um sicherzustellen, dass das Mittel (b) nach das Awendung nicht vom Keratinmaterial bzw. vom Haar heruntertropft, kann das Mittel (b) ferner zusätzlich ein Verdickundsmittel enthalten.

Im Rahmen einer weiteren besonders bevorzugten Ausführungsform ist ein erfindungsgemäßes Verfahren dadurch gekennzeichnet, dass das Mittel (b) mindestens einen Verdickungsmittel enthält.

Geeignete Verdickungsmittel umfassen beispielsweise chemisch modifizierte Cellulosen, wie beispielsweise Propylcellulose, Methylethylcellulose, Carboxymethylcellulose, Hydroxyethylcellulose, Methylhydroxyethylcellulose, Ethylhydroxyethylcellulose, Hydroxypropylcellulose, Hydroxypropylmethylcellulose, Carboxymethylhydroxyethylcellulose, Sulfoethylcellulose, Carboxymethylsulfoethylcellulose, Hydroxypropylsulfoethylcellulose, Hydroxyethylsulfoethylcellulose, Methylethylhydroxyethylcellulose, Methlylsulfoethylcellulose und/oder Ethylsulfoethylcellulose.

In einer bevorzugten Ausführungsform ist ein Verfahren zum Färben von keratinischem Material dadurch gekennzeichnet, dass das Mittel (b) ferner ein Verdickungsmittel ausgewählt aus der Gruppe bestehend aus Propylcellulose, Methylethylcellulose, Carboxymethylcellulose, Hydroxyethylcellulose, Methylhydroxyethylcellulose, Ethylhydroxyethylcellulose, Hydroxypropylcellulose, Hydroxypropylmethylcellulose, Carboxymethylhydroxyethylcellulose, Sulfoethylcellulose, Carboxymethylsulfoethylcellulose, Hydroxypropylsulfoethylcellulose, Hydroxyethylsulfoethylcellulose, Methylethylhydroxyethylcellulose, Methlylsulfoethylcellulose, Ethylsulfoethylcellulose und Mischungen daraus enthält.

Besonders geeignete Verdickungsmittel sind ausgewählt aus Hydroxyethylcellulose, Hydroxypropylcellulose, Hydroxypropylmethylcellulose und Mischungen daraus.

Weitere geeignete Verdickungsmittel umfassen Galaktomannane. Bevorzugte Galaktomannane umfassen Galaktomannane mit der INCI Bezeichnung Cyamopsis tetragonoloba gum (Guar Gum), Galaktomannane mit der INCI Bezeichnung Ceratonia Siliqua (Carob) Gum (Locust Bean Gum), Galaktomannane mit der INCI Bezeichnung Cassia Gum und Galaktomannane mit der INCI Bezeichnung Caesalpinia Spinosa Gum (Tara Gum).

Entsprechend ist ein Verfahren zum Färben von keratinischem Material besonders bevorzugt, bei dem das Mittel (b) ferner mindestens ein Galaktomannan enthält, welches ausgewählt ist aus der Gruppe bestehend aus Galaktomannanen mit der INCI Bezeichnung Cyamopsis tetragonoloba gum (Guar Gum), Galaktomannanen mit der INCI Bezeichnung Ceratonia Siliqua (Carob) Gum (Locust Bean Gum), Galaktomannanen mit der INCI Bezeichnung Cassia Gum und Galaktomannanen mit der INCI Bezeichnung Caesalpinia Spinosa Gum (Tara Gum) umfassen. In einer besonders bevorzugten Ausführungsform umfasst das Galaktomannan ein Galaktomannanen mit der INCI Bezeichnung Caesalpinia Spinosa Gum (Tara Gum).

Die Menge an Verdickungsmittel liegt bevorzugt zwischen 0,1 und 10 Gew.-% jeweils bezogen auf die Gesamtmenge an Mittel (b).

### Weitere Inhaltsstoffe in den Mitteln (a) und (b)

Die zuvor beschriebenen Mittel (a) und (b) können ferner auch noch ein oder mehrere optionale Inhaltsstoffe enthalten.

Bei den fakultativ in den Mitteln (a) und/oder (b) einsetzbaren kosmetischen Inhaltsstoffen kann es sich um alle geeigneten Bestandteile handeln, um dem Mittel weitere positive Eigenschaften zu verleihen. Beispielsweise können im Mittel (a) und/oder (b) zusätzlich eine oder mehrere oberflächenaktive Verbindungen aus der Gruppe der nichtionischen, kationischen, anionischen oder zwitterionischen/amphoteren Tenside enthalten sein.

Die Mittel können ferner auch noch weitere Wirk-, Hilfs- und Zusatzstoffe enthalten, wie beispielsweise Lösungsmittel, Fettbestandteile wie beispielsweise der C₈-C₃₀-Fettsäuretriglyceride, der C₈-C₃₀-Fettsäuremonoglyceride, der C₈-C₃₀-Fettsäurediglyceride und/oder der Kohlenwasserstoffe; Strukturanten wie Glucose, Maleinsäure und Milchsäure, haarkonditionierende Verbindungen wie Phospholipide, beispielsweise Lecithin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und - distearat sowie PEG-3-distearat; sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

### Schritt (3), Einwirkenlassen beider Mittel (a) und (b) auf das keratinische Material

Nach dem Vermischen der beiden Mittel (a) und (b) miteinander wird deren Gemisch auf all die Partien des Keratinmaterials einwirken gelassen, die mit dem Gemisch beider Mittel beaufschlagt sind.

Mit dem Vermischen der beiden Mittel (a) und (b) treten die im Mittel (a) enthaltenen Silane (a2) mit der im Mittel (b) vorhandenen definierten Wassermenge (b1) in Kontakt, so dass eine Hydrolyse und Oligomerisierung bzw. Polymerisierung der Silane (a2) initiiert wird. Die Ausbildung des Coatings bzw. Films erfolgt auf diese Weise an genau den Stellen des Keratinmaterials, die mit der Mischung beider Mittel (a) und (b) benetzt sind. Hierbei werden das Ausmaß und die Geschwindigkeit der Hydrolyse bzw. der Polymerisiation durch das Verhältnis der auf dem Haar befindlichen Silanmengen (a2) und Wassermengen (b1) bestimmt. Je kleiner das Mengenverhältnis von (a2) zu (b1), d.h. das Mengenverhältnis (a2)/(b1) ist, desto mehr Wasser steht für eine bestimmte Silanmenge zur Verfügung und desto schneller und umfassender läuft die Polymerisation ab. Das Mengenverhältnis (a2)/(b1) kann nun zum einen durch die Menge beider Mittel (a) und (b) bestimmt werden, die jeweils auf die Keratinmaterialien bzw. Haare aufgetragen werden. Weitere Einflussfaktoren sind die Konzentration der Silane (a2) im Mittel (a) und die Wasserkonzentration im Mittel (b). In diesem Zusammenhang hat sich herausgestellt, dass die Oligomerisierung bzw. Polymerisierung dann auf die für die Anwendung optimale Geschwindigkeit eingestellt werden konnte, wenn das Gewichtsverhältnis aus der Gesamtmenge der im Mittel (a) enthaltenen organischen Siliciumverbindungen (a2) zur Menge des im Mittel (b) enthaltenen Wassers (b1), d.h. das Gewichtsverhältnis (a2)/(b1), bei einem Wert von 1:100 bis 1:1, bevorzugt von 1:70 bis 1:2, weiter bevorzugt von 1:70 bis 1:5 und ganz besonders bevorzugt von 1:70 bis 1:10 liegt.

In anderen Worten konnte dann ein besonders gleichmäßiger und widerstandsfähiger Film auf dem Keratinmaterial erzeugt werden, wenn sowohl die Mengen der Mittel (a) und (b) als auch die Konzentrationen der Silane (a2) und des Wassers (b1) in beiden Mitteln so gewählt wurden, dass die Silane (a2) und das Wasser (b1) in einem Gewichtsverhältnis von 1:100 bis 1:1, bevorzugt von 1:70 bis 1:2, weiter bevorzugt von 1:70 bis 1:5 und ganz besonders bevorzugt von 1:70 bis 1:10 auf dem Keratinmaterial vorlagen.

Ein Gewichtsverhältnis (a2)/(b1) von 1:70 bis 1:10 bedeutet hierbei, dass das aus dem Mittel (b) stammende Wasser (b1) im Vergleich zur Gesamtmenge der aus dem Mittel (a) stammenden Silane (a2) ein einem 10-fachen bis 70-fachen Gewichtsüberschuss eingesetzt wird.

### Beispiel

40 g des Mittels (a) werden auf die Haare eines Probanden aufgetragen, das Mittel (a) wird kurz in die gesamte Haarmasse einmassiert.

Das Mittel (a) enthält
1,0 Gew.-% Wasser (a1) (bzw. ein Gemisch aus Wasser und Säure oder ein Gemisch aus Wasser und Lauge),
3,0 Gew.-% 3-(Aminopropyl)-triethoxysilan (a2),
6,0 Gew.-% Methyltriethoxysilan (a2),
1,0 Gew.-% Pigment (z.B. Unipure Red LC 3079),
89 Gew.-% Polyethylenglycol (z.B. PEG-8).

Die Gesamtmenge der im Mittel (a) enthaltenen Silane (a2) beträgt 3,6 g, d.h. im Mittel (a) sind 1,2 g 3-(Aminopropyl)-triethoxysilan sowie 2,4 g Methyltriethoxysilan enthalten.

Im Anschluss an die Applikation des Mittels (a) werden nun 100 g des Mittels (b) aufgetragen. Bei dem Mittel (b) handelt es sich um eine angedickte wässrige Lösung mit einem Wassergehalt von 99 Gew.-%. Das Mittel (b) wird ebenfalls gleichmäßig auf dem gesamten Kopf des Probanden verteilt.

Nach der Anwendung des Mittels (b) werden die Haare kräftig für 1 Minute mit den Fingern massiert, so dass beide Mittel (a) und (b) vollständig miteinander vermischt werden.

Im Mittel (b) sind 99 g Wasser (b1) vorhanden.

Das Gewichtsverhältnis aus der Gesamtmenge der im Mittel (a) enthaltenen organischen Siliciumverbindungen (a2) zur Menge des im Mittel (b) enthaltenen Wassers (b1), d.h. das Gewichtsverhältnis (a2)/(b1), beträgt 3,6 : 99 = 1 : 27,5.

Im Rahmen einer explizit ganz besonders bevorzugten Ausführungsform ist ein erfindungsgemäßes Verfahren dadurch gekennzeichnet, dass das Gewichtsverhältnis aus der Gesamtmenge der im Mittel (a) enthaltenen organischen Siliciumverbindungen (a2) zur Menge des im Mittel (b) enthaltenen Wassers (b1), d.h. das Gewichtsverhältnis (a2)/(b1), bei einem Wert von 1:100 bis 1:1, bevorzugt von 1:70 bis 1:2, weiter bevorzugt von 1:70 bis 1:5 und ganz besonders bevorzugt von 1:70 bis 1:10 liegt.

Nach dem Vermischen der beiden Mittel (a) und (b) werden nun beide Mittel gemeinsam in Form ihres Gemisches auf das Keratinmaterial einwirken gelassen. Bevorzugt werden beide Mittel (a) und (b) gemeinsam für einen Zeitraum von 10 Sekunden bis 30 Minuten, bevorzugt von 30 Sekunden bis 20 Minuten, weiter bevorzugt von 1 Minute bis 15 Minuten und ganz besonders von 5 Minuten bis 10 Minuten auf dem Keratinmaterial, inbesondere auf die menschlichen Haare, einwirken gelassen.

Im Rahmen einer weiteren bevorzugten Ausführungsform ist ein erfindungsgemäßes Verfahren gekennzeichnet durch das
(3) gemeinsame Einwirkenlassen beider Mittel (a) und (b) auf das keratinische Material für einen Zeitraum von 10 Sekunden bis 30 Minuten, bevorzugt von 30 Sekunden bis 20 Minuten, weiter bevorzugt von 1 Minute bis 15 Minuten und ganz besonders von 5 Minuten bis 10 Minuten.

### Schritt (4), Ausspülen beider Mittel (a) und (b)

Die zu dieser Erfindung führenden Arbeiten haben gezeigt, dass sich nach Ablauf der Einwirkzeit in Schritt (3) des Verfahrens ein gefärbter Film ausgebildet hat, der sich durch besonders hohe Gleichmäßigkeit auszeichnete. Bei Anwendung auf Haaren in Form einer Ganzkopfanwendung konnte so der gesamte Kopf mit ebenmäßigem Farbergebnis gefärbt werden. Nach dem Einwirken der beiden Mittel (a) und (b) konnten diese dann vom Keratinmaterial wieder abgespült bzw. aus den Haaren ausgespült werden.

Schritt (4) des ErfindungsgemäßenVerfahrens umfasst daher das Ausspülen beider Mittel (a) und (b). Das Ausspülen der beiden Mittel (a) und (b) erfolgt hierbei bevorzugt unter fließendem Wasser.

Im Rahmen einer weiteren bevorzugten Ausführungsform ist ein erfindungsgemäßes Verfahren gekennzeichnet durch das
(4) Ausspülen beider Mittel (a) und (b) unter fließendem Wasser.

### Beispiele

Es wurden die folgenden Formulierungen hergestellt (sofern nichts anderes angegeben ist, sind alle Angaben in Gew.-%)

### Mittel (a)

| Mittel (a) | Gew.-% |
|---|---|
| (3-Aminopropyl)triethoxysilan (a2) | 3,0 |
| Methyltriethoxysilan (a2) | 6,0 |
| Wasser (a1) | 1,0 |
| NaOH | 0,01 |
| Unipure Red LC 3079 (Pigment Red 7, CAS-Nr. 5281-04-9) (a3) | 1,0 |
| PEG-8 (Polyethylenglycol, Molgewicht 400 g/mol) | ad 100 |

### Mittel (b)

| Mittel (b) | Gew.-% |
|---|---|
| Wasser | 99,0 |
| Hyroxyethylcellulose (Natrosol 250 HR) | 1,0 |

5 Haarsträhnen (Kerling naturweiß) wurden unter laufendem Wasser angefeuchtet und anschließend für 30 Sekunden mit einem Handtuch trockengerubbelt. Dann wurde das Mittel (a) auf jede handtuchtrockene Haarsträhne appliziert (0,4 g Mittel (a) pro Haarsträhne). Das Mittel (a) wurde für 30 Sekunden in jede Haarsträhne einmassiert. Direkt im Anschluss daran wurde das Mittel (b) auf die Haarsträhnen aufgetagen (1,0 g Mittel (b) pro Haarsträhne). Jede Haarsträhne wurde nochmals für 30 Sekunden massiert, so dass sich eine Mischung der Mittel (a) und (b) ausbildete.

Auf das Haar wurden 0,036 g Silane (a2) aufgetragen.

Aus das Haar wurden 0,99 g Wasser (b1) aufgetragen.

Das Gewichtsverhältnis (a2)/(b1) betrug 0,036 : 0,99 = 1 : 27,5

Diese Mischung der Mittel aus (a) und (b) wurde für 5 Minuten auf die Haarsträhnen einwirken gelassen. Danach wurde die Haarsträhne unter fließendem Wasser ausgespült und getrocknet. Es wurde eine gleichmäßige und intensive Färbung erhalten. Hierbei hatten alle 5 Haarsträhnen die gleiche rote Färbung und besaßen eine gleich hohe Farbintensität.

### Vergleich

5 Haarsträhnen (Kerling naturweiß) wurden unter laufendem Wasser angefeuchtet und anschließend für 30 Sekunden mit einem Handtuch trockengerubbelt._Dann wurden 40 g des Mittels (a) mit 100 g des Mittels (b) vermischt. Diese Anwendungsmischung wurde auf 5 Haarsträhnen aufgetragen (jeweils 1,4 g Anwendungsmischung pro Haarsträhne). Jede Haarsträhne wurde für 30 Sekunden massiert. Anschließend wurde die Anwendungsmischung jeweils für 5 Minuten auf jede Haarsträhnen einwirken gelassen. Dann wurde die Haarsträhne unter fließendem Wasser ausgespült und getrocknet. Alle Haarsträhnen waren rot gefärbt. Die Haarsträhne, die zuerst gefärbt wurde, besaß jedoch eine höhere Farbintensität als die Haarsträhne, die zuletzt mit der Mischung aus (a) und (b) behandelt wurde.

## Patentansprüche

1. Verfahren zum Färben von keratinischem Material, insbesondere menschlichen Haaren, umfassend die folgenden Schritte in der angegebenen Reihenfolge:
(1) Anwendung eines Mittels (a) auf dem keratinischem Material, wobei das Mittel (a) - bezogen auf das Gesamtgewicht des Mittels (a) - enthält:
(a1) weniger als 10 Gew.-% Wasser,
(a2) mindestens eine organische Siliciumverbindung aus der Gruppe der Silane mit einem, zwei oder drei Siliciumatomen, und
(a3) mindestens ein farbgebende Verbindung,
(2) Anwendung eines Mittels (b) auf dem keratinischem Material, das noch mit dem Mittel (a) beaufschlagt ist, wobei das Mittel (b) enthält:
(b1) Wasser
(3) Einwirkenlassen beider Mittel (a) und (b) auf das keratinische Material, und
(4) Ausspülen beider Mittel (a) und (b).

2. Verfahren nach Anspruch 1, **dadurch gekennzeichnet, dass** das Mittel (a) in Schritt (1) auf handtuchtrockenem oder trockenem keratinischen Material angewendet wird.

3. Verfahren nach einem der Ansprüche 1 bis 2, **dadurch gekennzeichnet, dass** das Mittel (a) - bezogen auf das Gesamtgewicht des Mittels (a) - von 0 bis 7,5 Gew.-%, bevorzugt von 0,0 bis 5,0 Gew.-%, weiter bevorzugt von 0,0 bis 4,0 Gew.-% und ganz besonders bevorzugt 0,1 bis 2,5 Gew.-% Wasser (a1) enthält.

4. Verfahren nach einem der Ansprüche 1 bis 3, **dadurch gekennzeichnet, dass** das Mittel (a) mindestens eine organische Siliciumverbindung (a2) der Formel (I) und/oder (II) enthält
R₁R₂N-L-Si(OR₃)ₐ(R₄)_{b} (I),
wobei
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃, R₄ unabhängig voneinander für eine C₁-C₆-Alkylgruppe stehen,
- a, für eine ganze Zahl von 1 bis 3 steht, und
- b für die ganze Zahl 3 - a steht, und
wobei in der organischen Siliciumverbindung der Formel (II)
(R₅O)_{c}(Rₑ)_{d}Si-(A)ₑ-[NR₇-(A')]_{f}-[O-(A")]_{g}-[NR₈-(A‴)]ₕ-Si(R₆')_{d'}(OR₅')_{c}· (II),
- R5, R5', R5", R6, R6' und R6" unabhängig voneinander für eine C₁-C₆-Alkylgruppe stehen,
- A, A', A", A‴ und A‴' unabhängig voneinander für eine lineare oder verzweigte, zweiwertige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkylgruppe oder eine Gruppierung der Formel (III) stehen
-
(Aʺʺ)-Si(R₆")_{d}"(OR₅")_{c}" (III)
,
- c, für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
mit der Maßgabe, dass mindestens einer der Reste aus e, f, g und h von 0 verschieden ist.

5. Verfahren nach einem der Ansprüche 1 bis 4, **dadurch gekennzeichnet, dass** das Mittel (a) mindestens eine organische Siliciumverbindung (a2) der Formel (I) enthält,
R₁R₃N-L-Si(OR₃)ₐ(R₄)_{b} (I),
wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht,
- R₃, R₄ unabhängig voneinander für eine Methylgruppe oder für eine Ethylgruppe stehen,
- a für die Zahl 3 steht und
- b für die Zahl 0 steht.

6. Verfahren nach einem der Ansprüche 1 bis 5, **dadurch gekennzeichnet, dass** das Mittel (a) mindestens eine organische Siliciumverbindung (a2) enthält, die ausgewählt ist aus der Gruppe aus
- (3-Aminopropyl)triethoxysilan
- (3-Aminopropyl)trimethoxysilan
- 1-(3-Aminopropyl)silantriol
- (2-Aminoethyl)triethoxysilan
- (2-Aminoethyl)trimethoxysilan
- 1-(2-Aminoethyl)silantriol
- (3-Dimethylaminopropyl)triethoxysilan
- (3-Dimethylaminopropyl)trimethoxysilan
- 1-(3-Dimethylaminopropyl)silantriol
- (2-Dimethylaminoethyl)triethoxysilan.
- (2-Dimethylaminoethyl)trimethoxysilan und/oder
- 1-(2-Dimethylaminoethyl)silantriol.

7. Verfahren nach einem der Ansprüche 1 bis 6, **dadurch gekennzeichnet, dass** das Mittel (a) mindestens eine organische Siliciumverbindung (a2) der Formel (IV) enthält.
R₉Si(OR₁₀)ₖ(R₁₁)ₘ (IV),
wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

8. Verfahren nach einem der Ansprüche 1 bis 7, **dadurch gekennzeichnet, dass** das Mittel (a) mindestens eine organische Siliciumverbindung (a2) enthält, die ausgewählt ist aus der Gruppe aus
- Methyltrimethoxysilan
- Methyltriethoxysilan
- Ethyltrimethoxysilan
- Ethyltriethoxysilan
- Propyltrimethoxysilan
- Propyltriethoxysilan
- Hexyltrimethoxysilan
- Hexyltriethoxysilan
- Octyltrimethoxysilan
- Octyltriethoxysilan
- Dodecyltrimethoxysilan,
- Dodecyltriethoxysilan,
- Octadecyltrimethoxysilan und
- Octadecyltriethoxysilan.

9. Verfahren nach einem der Ansprüche 1 bis 8, **dadurch gekennzeichnet, dass** das Mittel (a) mindestens zwei strukturell voneinander verschiedene organische Siliciumverbindungen (a2) enthält.

10. Verfahren nach einem der Ansprüche 1 bis 9, dadurchgekennzeichnet, dass das Mittel (a) - bezogen auf das Gesamtgewicht des Mittels (a) - eine oder mehrere organische Siliciumverbindungen (a2) in einer Gesamtmenge von 0,1 bis 20 Gew.-%, bevorzugt 1 bis 15 Gew.-% und besonders bevorzugt 2 bis 8 Gew.-% enthält.

11. Verfahren nach einem der Ansprüche 1 bis 10, **dadurch gekennzeichnet, dass** das Mittel (a) mindestens eine farbgebende Verbindung (a3) aus der Gruppe aus Pigmenten und/oder direktziehenden Farbstoffen enthält.

12. Verfahren nach einem der Ansprüche 1 bis 11, **dadurch gekennzeichnet, dass** das Mittel (a) mindestens ein anorganisches Pigment (a3) enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

13. Verfahren nach einem der Ansprüche 1 bis 12, **dadurch gekennzeichnet, dass** das Mittel (a) mindestens ein organisches Pigment (a3) enthält, das bevorzugt ausgewählt ist aus der Gruppe aus Carmin, Chinacridon, Phthalocyanin, Sorgho, blauen Pigmenten mit den Color Index Nummern CI 42090, CI 69800, CI 69825, CI 73000, CI 74100, CI 74160, gelben Pigmenten mit den Color Index Nummern CI 11680, CI 11710, CI 15985, CI 19140, CI 20040, CI 21100, CI 21108, CI 47000, CI 47005, grünen Pigmenten mit den Color Index Nummern CI 61565, CI 61570, CI 74260, orangen Pigmenten mit den Color Index Nummern CI 11725, CI 15510, CI 45370, CI 71105, roten Pigmenten mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und CI 75470.

14. Verfahren nach einem der Ansprüche 1 bis 13, **dadurch gekennzeichnet, dass** das Mittel (a) ein oder mehrere farbgebende Verbindungen (a3) aus der Gruppe der Pigmente auf Basis eines lamellaren Substratplättchens, der Pigmente auf Basis eines lentikularen Substratplättchens und der Vakuum metallisierten Pigmente enhält.

15. Verfahren nach einem der Ansprüche 1 bis 14, **dadurch gekennzeichnet, dass** das Mittel (a) mindestens einen kosmetischen Träger (a4) aus der Gruppe aus Poly-C₁-C₆-Alkylenglycolen, 1,2-Propylenglycol, 1,3-Propylenglycol, 1,2-Butylenglycol, Dipropylenglycol, Ethanol, Isopropanol, Diethylenglycolmonoethylether, Glycerin, Phenoxyethanol und Benzylalkohol, ganz besonders bevorzugt aus Polyethylenglycolen, enthält.

16. Mittel nach einem der Ansprüche 1 bis 15, **dadurch gekennzeichnet, dass** das Mittel (a) - bezogen auf das Gesamtgewicht des Mittels (a) - ein oder mehrere Bestandteile des kosmetischen Trägers (a4) in einer Gesamtmenge von 10,0 bis 99,0 Gew.-%, bevorzugt von 30,0 bis 99,0 Gew.-%, weiter bevorzugt von 50,0 bis 99,0 Gew.-% und ganz besonders bevorzugt von 70,0 bis 99,0 Gew.-% enthält.

17. Verfahren nach einem der Ansprüche 1 bis 16, **gekennzeichnet durch** die Anwendung des Mittels (b) innerhalb von 10 Minuten, bevorzugt innerhalb von 5 Minuten nach Anwendung des Mittels (a).

18. Verfahren nach einem der Ansprüche 1 bis 17, **dadurch gekennzeichnet**, durch die
(2) Anwendung eines Mittels (b) auf dem keratinischem Material, das noch mit dem Mittel (a) beaufschlagt ist, und Herstellung einer Mischung aus beiden Mitteln (a) und (b) auf dem Keratinmaterial durch manuelles Vermischen beider Mittel.

19. Verfahren nach einem der Ansprüche 1 bis 18, **dadurch gekennzeichnet, dass** das Mittel (b) - bezogen auf das Gesamtgewicht des Mittels (b) - 10 bis 100 Gew.-%, bevorzugt 30 bis 99,5 Gew.-%, weiter bevorzugt 50 bis 99 Gew.-% und ganz besonders bevorzugt 80 bis 99 Gew.-% Wasser enthält.

20. Verfahren nach einem der Ansprüche 1 bis 19, **dadurch gekennzeichnet, dass** das Mittel (b) mindestens ein Verdickungsmittel enthält.

21. Verfahren nach einem der Ansprüche 1 bis 20, **dadurch gekennzeichnet, dass** das Gewichtsverhältnis aus der Gesamtmenge der im Mittel (a) enthaltenen organischen Siliciumverbindungen (a2) zur Menge des im Mittel (b) enthaltenen Wassers (b1), d.h. das Gewichtsverhältnis (a2)/(b1), bei einem Wert von 1:100 bis 1:1, bevorzugt von 1:70 bis 1:2, weiter bevorzugt von 1:70 bis 1:5 und ganz besonders bevorzugt von 1:70 bis 1:10 liegt.

22. Verfahren nach einem der Ansprüche 1 bis 21, **gekennzeichnet durch** das
(3) gemeinsame Einwirkenlassen beider Mittel (a) und (b) auf das keratinische Material für einen Zeitraum von 10 Sekunden bis 30 Minuten, bevorzugt von 30 Sekunden bis 20 Minuten, weiter bevorzugt von 1 Minute bis 15 Minuten und ganz besonders von 5 Minuten bis 10 Minuten.

23. Verfahren nach einem der Ansprüche 1 bis 22, **gekennzeichnet durch** das
(4) Ausspülen beider Mittel (a) und (b) unter fließendem Wasser.

## Claims

1. Method for dyeing keratinous material, in particular human hair, comprising the following steps in the order indicated:
(1) applying an agent (a) to the keratinous material, wherein the agent (a) contains, based on the total weight of the agent (a):
(a1) less than 10% by weight of water,
(a2) at least one organic silicon compound from the group of silanes with one, two or three silicon atoms, and
(a3) at least one coloring compound,
(2) application of an agent (b) to the keratinous material still coated with the agent (a), wherein the agent (b) contains:
(b1) water
(3) Allowing both agents (a) and (b) to act on the keratinous material, and
(4) Rinsing off both agents (a) and (b).

2. Method according to claim 1, **characterized in that** the agent (a) is applied in step (1) to toweldried or dry keratinous material.

3. Method according to one of claims 1 to 2, **characterized in that** the agent (a) contains 0 to 7.5% by weight, preferably 0.0 to 5.0% by weight, more preferably 0.0 to 4.0% by weight and most preferably 0.1 to 2.5% by weight of water (a1), based on the total weight of the agent (a).

4. Method according to one of claims 1 to 3, **characterized in that** the agent (a) contains at least one organic silicon compound (a2) of formula (1) and/or (II)
R₁R₂N-L-Si(OR₃)ₐ(R₄ )_{b} (I),
where
- R₁ , R₂ independently of one another represent a hydrogen atom or a C₁ -C₆ alkyl group,
- L represents a linear or branched, divalent C₁ -C₂₀ -alkylene group,
- R₃ , R₄ independently of one another represent a C₁ -C₆ -alkyl group,
- a is an integer from 1 to 3, and
- b stands for the integer 3 - a, and
wherein, in the organic silicon compound of formula (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R5, R5', R5", R6, R6' and R6" independently represent a C₁-C₆alkyl group,
- A, A', A", A‴, and A‴' independently represent a linear or branched, divalent C₁ -C₂₀ - alkylene group,
- R₇ and R₈ independently of one another represent a hydrogen atom, a C₁ -C₆ -alkyl group, a hydroxy-C₁ -C₆ -alkyl group, a C₂ -C₆ -alkenyl group, an amino-C₁ -C₆ -alkyl group or a group of formula (III)
-
(A‴')-Si(R₆")_{d}"(OR₅")_{c}" (III),
- c stands for an integer from 1 to 3,
- d stands for the integer 3 - c,
- c' stands for an integer from 1 to 3,
- d' stands for the integer 3 - c',
- c" stands for an integer from 1 to 3,
- d" stands for the integer 3 - c",
- e stands for 0 or 1,
- f stands for 0 or 1,
- g stands for 0 or 1,
- h stands for 0 or 1,
with the proviso that at least one of the residues from e, f, g and h is different from 0.

5. Method according to one of claims 1 to 4, **characterized in that** the agent (a) contains at least one organic silicon compound (a2) of formula (I)
R₁R₂N-L-Si(OR₃)ₐ(R₄)_{b} (I),
where
- R₁ , R₂ both represent a hydrogen atom, and
- L represents a linear, divalent C₁ -C₆ alkylene group, preferably a propylene group (-CH₂ -CH₂ -CH₂ -) or an ethylene group (-CH₂ -CH₂-),
- R₃ , R₄ independently represent a methyl group or an ethyl group,
- a stands for the number 3 and
- b stands for the number 0.

6. Method according to any of claims 1 to 5, **characterized in that** the agent (a) contains at least one organic silicon compound (a2) selected from the group consisting of
- (3-aminopropyl)triethoxysilane
- (3-aminopropyl)trimethoxysilane
- 1-(3-aminopropyl)silantriol
- (2-aminoethyl)triethoxysilane
- (2-aminoethyl)trimethoxysilane
- 1-(2-Aminoethyl)silantriol
- (3-Dimethylaminopropyl)triethoxysilane
- (3-Dimethylaminopropyl)trimethoxysilane
- 1-(3-dimethylaminopropyl)silantriol
- (2-Dimethylaminoethyl)triethoxysilane.
- (2-Dimethylaminoethyl)trimethoxysilane and/or
- 1-(2-dimethylaminoethyl)silane triol.

7. Process according to one of claims 1 to 6, **characterized in that** the agent (a) contains at least one organic silicon compound (a2) of formula (IV).
R₉Si(OR₁₀)ₖ(R₁₁)ₘ (IV),
where
- R₉ represents a C₁ -C₁₈ alkyl group,
- R₁₀ represents a hydrogen atom or a C₁ -C₆ -alkyl group,
- R₁₁ represents a C₁ -C₆ -alkyl group
- k stands for an integer from 1 to 3, and
- m stands for the integer 3 - k.

8. Method according to any of claims 1 to 7, **characterized in that** the agent (a) contains at least one organic silicon compound (a2) selected from the group consisting of
- methyltrimethoxysilane
- methyltriethoxysilane
- ethyl trimethoxysilane
- Ethyltriethoxysilane
- propyltrimethoxysilane
- Propyltriethoxysilane
- Hexyltrimethoxysilane
- Hexyltriethoxysilane
- Octyltrimethoxysilane
- Octyltriethoxysilane
- Dodecyltrimethoxysilane,
- dodecyltriethoxysilane,
- octadecyltrimethoxysilane, and
- octadecyltriethoxysilane.

9. Method according to one of claims 1 to 8, **characterized in that** the agent (a) contains at least two structurally different organic silicon compounds (a2).

10. Method according to one of claims 1 to 9, **characterized in that** the agent (a) contains one or more organic silicon compounds (a2) in a total amount of 0.1 to 20 wt.%, preferably 1 to 15 wt.% and particularly preferably 2 to 8 wt.%, based on the total weight of the agent (a).

11. Method according to one of claims 1 to 10, **characterized in that** the agent (a) contains at least one coloring compound (a3) from the group consisting of pigments and/or direct dyes.

12. Method according to one of claims 1 to 11, **characterized in that** the agent (a) contains at least one inorganic pigment (a3) which is preferably selected from the group consisting of colored metal oxides, metal hydroxides, metal oxide hydrates, silicates, metal sulfides, complex metal cyanides, metal sulfates, bronze pigments, and/or colored pigments based on mica or micaceous substances coated with at least one metal oxide and/or a metal oxychloride.

13. Method according to one of claims 1 to 12, **characterized in that** the agent (a) contains at least one organic pigment (a3) which is preferably selected from the group consisting of carmine, quinacridone, phthalocyanine, sorghum, blue pigments with the color index numbers CI 42090, CI 69800, CI 69825, CI 73000, CI 74100, CI 74160, yellow pigments with Color Index numbers Cl 11680, Cl 11710, CI 15985, Cl 19140, Cl 20040, Cl 21100, Cl 21108, Cl 47000, Cl 47005, green pigments with Color Index numbers Cl 61565, Cl 61570, Cl 74260, orange pigments with Color Index numbers Cl 11725, CI 15510, Cl 45370, Cl 71105, red pigments with Color Index numbers CI 12085, Cl 12120, Cl 12370, CI 12420, Cl 12490, Cl 14700, Cl 15525, CI 15580, Cl 15620, Cl 15630, Cl 15800, CI 15850, CI 15865, Cl 15880, Cl 17200, Cl 26100, CI 45380, CI 45410, CI 58000, Cl 73360, Cl 73915 and Cl 75470.

14. Method according to one of claims 1 to 13, **characterized in that** the agent (a) contains one or more coloring compounds (a3) from the group of pigments based on a lamellar substrate plate, pigments based on a lenticular substrate plate and vacuum-metallized pigments.

15. Method according to one of claims 1 to 14, **characterized in that** the agent (a) contains at least one cosmetic carrier (a4) from the group consisting of poly-C₁ -C₆ -alkylene glycols, 1,2-propylene glycol, 1,3-propylene glycol, 1,2-butylene glycol, dipropylene glycol, ethanol, isopropanol, diethylene glycol monoethyl ether, glycerin, phenoxyethanol and benzyl alcohol, particularly preferred from polyethylene glycols.

16. Agent according to one of claims 1 to 15, **characterized in that** the agent (a) - based on the total weight of the agent (a) - contains one or more components of the cosmetic carrier (a4) in a total amount of 10.0 to 99.0 wt.%, preferably 30.0 to 99.0 wt.%, more preferably from 50.0 to 99.0% by weight and most preferably from 70.0 to 99.0% by weight.

17. Method according to one of claims 1 to 16, **characterized by** the application of agent (b) within 10 minutes, preferably within 5 minutes after application of agent (a).

18. Method according to one of claims 1 to 17, **characterized by** the
(2) application of an agent (b) to the keratin material still coated with the agent (a) and production of a mixture of both agents (a) and (b) on the keratin material by manually mixing both agents.

19. Method according to one of claims 1 to 18, **characterized in that** the agent (b) - based on the total weight of the agent (b) - contains 10 to 100 wt.%, preferably 30 to 99.5 wt.%, more preferably 50 to 99 wt.% and most preferably 80 to 99 wt.% water.

20. Method according to one of claims 1 to 19, **characterized in that** the agent (b) contains at least one thickening agent.

21. Method according to one of claims 1 to 20, **characterized in that** the weight ratio of the total amount of organic silicon compounds (a2) contained in agent (a) to the amount of water (b1) contained in agent (b), i.e. the weight ratio (a2)/(b1), is between 1:100 and 1:1, preferably between 1:70 and 1:2, more preferably between 1:70 and 1:5, and most preferably between 1:70 and 1:10.

22. Method according to one of claims 1 to 21, **characterized by**
(3) allowing both agents (a) and (b) to act together on the keratinous material for a period of 10 seconds to 30 minutes, preferably 30 seconds to 20 minutes, more preferably 1 minute to 15 minutes, and most preferably 5 minutes to 10 minutes.

23. Method according to any of claims 1 to 22, **characterized by**
(4) Rinsing both agents (a) and (b) under running water.

## Revendications

1. Procédé de coloration de matière kératinique, en particulier de cheveux humains, comprenant les étapes suivantes dans l'ordre indiqué :
(1) application d'un agent (a) sur la matière kératinique, l'agent (a) contenant, par rapport au poids total de l'agent (a) :
(a1) moins de 10 % en poids d'eau,
(a2) au moins un composé organique du silicium choisi dans le groupe des silanes comportant un, deux ou trois atomes de silicium, et
(a3) d' t au moins un composé colorant,
(2) application d'un agent (b) sur la matière kératinique encore imprégnée de l'agent (a), l'agent (b) contenant :
(b1) de l'eau
(3) laisser agir les deux agents (a) et (b) sur la matière kératinique, et
(4) rinçage des deux agents (a) et (b).

2. Procédé selon la revendication 1, **caractérisé en ce que** l'agent (a) est appliqué à l'étape (1) sur une matière kératinique sèche ou séchée à l'aide d'une serviette.

3. Procédé selon l'une des revendications 1 à 2, **caractérisé en ce que** l'agent (a) contient, par rapport au poids total de l'agent (a), de 0 à 7,5 % en poids, de préférence de 0,0 à 5,0 % en poids, de préférence encore de 0,0 à 4,0 % en poids et de manière particulièrement préférée de 0,1 à 2,5 % en poids d'eau (a1).

4. Procédé selon l'une des revendications 1 à 3, **caractérisé en ce que** le composé (a) contient au moins un composé organique du silicium (a2) de formule (I) et/ou (II)
R₁R₂N-L-Si(OR₃)ₐ(R₄)_{b} (I),
où
- R₁, R₂ représentent indépendamment l'un de l'autre un atome d'hydrogène ou un groupe alkyle en C₁ -C₆,
- L représente un groupe alkylène C₁ -C₂₀ divalent, linéaire ou ramifié,
- R₃ et R₄ représentent indépendamment l'un de l'autre un groupe alkyle en C₁ -C₆,
- a représente un nombre entier compris entre 1 et 3, et
- b représente le nombre entier 3 - a, et
où, dans le composé organique de silicium de formule (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R5, R5', R5", R6, R6' et R6" représentent indépendamment les uns des autres un groupe alkyle en C₁ -C₆,
- A, A', A", A‴ et Aʺʺ représentent indépendamment les uns des autres un groupe alkylène C₁ -C₂₀ divalent, linéaire ou ramifié,
- R₇ et R₈ représentent indépendamment l'un de l'autre un atome d'hydrogène, un groupe alkyle ₑₙ C₁ -C₆, un groupe hydroxy-C₁ -C₆ -alkyle, un groupe C₂ -C₆ -alcényle, un groupe amino-C₁ -C₆ -alkyle ou un groupement de formule (III)
-
(Aʺʺ)-Si(R₆")_{d}"(OR₅")_{c}" (III),
- c représente un nombre entier compris entre 1 et 3,
- d représente le nombre entier 3 - c,
- c' représente un nombre entier compris entre 1 et 3,
- d' représente le nombre entier 3 - c',
- c" représente un nombre entier compris entre 1 et 3,
- d" représente le nombre entier 3 - c",
- e représente 0 ou 1,
- f représente 0 ou 1,
- g représente 0 ou 1,
- h représente 0 ou 1,
à condition qu'au moins l'un des restes e, f, g et h soit différent de 0.

5. Procédé selon l'une des revendications 1 à 4, **caractérisé en ce que** le composé (a) contient au moins un composé organique du silicium (a2) de formule (I)
R₁R₂N-L-Si(OR₃)ₐ(R₄)_{b} (I),
où
- R₁ , R₂ représentent tous deux un atome d'hydrogène, et
- L représente un groupe alkylène linéaire divalent ₑₙ C₁ -C₆, de préférence un groupe propylène (-CH₂ -CH₂-CH₂-) ou un groupe éthylène (-CH₂ -CH₂-),
- R₃ , R₄ représentent indépendamment l'un de l'autre un groupe méthyle ou un groupe éthyle,
- a représente le chiffre 3 et
- b représente le chiffre 0.

6. Procédé selon l'une des revendications 1 à 5, **caractérisé en ce que** l'agent (a) contient au moins un composé organique de silicium (a2) choisi dans le groupe constitué par
- (3-aminopropyl)triéthoxysilane
- (3-aminopropyl)triméthoxysilane
- 1-(3-aminopropyl)silantriol
- (2-aminoéthyl)triéthoxysilane
- (2-aminoéthyl)triméthoxysilane
- 1-(2-aminoéthyl)silantriol
- (3-diméthylaminopropyl)triéthoxysilane
- (3-diméthylaminopropyl)triméthoxysilane
- 1-(3-diméthylaminopropyl)silantriol
- (2-diméthylaminoéthyl)triéthoxysilane.
- (2-diméthylaminoéthyl)triméthoxysilane et/ou
- 1-(2-diméthylaminoéthyl)silantriol.

7. Procédé selon l'une des revendications 1 à 6, **caractérisé en ce que** le composé (a) contient au moins un composé organique du silicium (a2) de formule (IV).
R₉Si(OR₁₀)ₖ(R₁₁)ₘ (IV),
dans laquelle
- R₉ représente un groupe alkyle en C₁ à C₁₈,
- R₁₀ représente un atome d'hydrogène ou un groupe alkyle en C₁ -C₆,
- R₁₁ représente un groupe alkyle en C₁ -C₆
- k représente un nombre entier compris entre 1 et 3, et
- m représente le nombre entier 3 - k.

8. Procédé selon l'une des revendications 1 à 7, **caractérisé en ce que** le composé (a) contient au moins un composé organique du silicium (a2) choisi dans le groupe constitué par
- méthyltriméthoxysilane
- méthyltriéthoxysilane
- éthyl triméthoxysilane
- éthyl triéthoxysilane
- propyltriméthoxysilane
- Propyltriéthoxysilane
- Hexyltriméthoxysilane
- Hexyltriéthoxysilane
- Octyltriméthoxysilane
- Octyltriéthoxysilane
- Dodécyltriméthoxysilane,
- dodécyltriéthoxysilane,
- octadécyltriméthoxysilane et
- octadécyltriéthoxysilane.

9. Procédé selon l'une des revendications 1 à 8, **caractérisé en ce que** l'agent (a) contient au moins deux composés organiques de silicium (a2) de structure différente.

10. Procédé selon l'une des revendications 1 à 9, **caractérisé en ce que** l'agent (a) contient, par rapport au poids total de l'agent (a), un ou plusieurs composés organiques de silicium (a2) en une quantité totale de 0,1 à 20 % en poids, de préférence de 1 à 15 % en poids et de manière particulièrement préférée de 2 à 8 % en poids.

11. Procédé selon l'une des revendications 1 à 10, **caractérisé en ce que** l'agent (a) contient au moins un composé colorant (a3) choisi dans le groupe constitué par les pigments et/ou les colorants directs.

12. Procédé selon l'une des revendications 1 à 11, **caractérisé en ce que** le produit (a) contient au moins un pigment inorganique (a3) choisi de préférence dans le groupe des oxydes métalliques colorés, des hydroxydes métalliques, des hydrates d'oxydes métalliques, des silicates, sulfures métalliques, cyanures métalliques complexes, sulfates métalliques, pigments bronze et/ou pigments colorés à base de mica ou de mica recouverts d'au moins un oxyde métallique et/ou un oxychlorure métallique.

13. Procédé selon l'une des revendications 1 à 12, **caractérisé en ce que** le produit (a) contient au moins un pigment organique (a3) choisi de préférence dans le groupe constitué par le carmin, la quinacridone, la phtalocyanine, le sorgho, les pigments bleus ayant les numéros d'index de couleur CI 42090, Cl 69800, Cl 69825, CI 73000, CI 74100, Cl 74160, des pigments jaunes ayant les numéros d'index de couleur CI 11680, CI 11710, CI 15985, CI 19140, CI 20040, CI 21100, CI 21108, CI 47000, CI 47005, des pigments verts portant les numéros d'index de couleur CI 61565, CI 61570, CI 74260, des pigments orange portant les numéros d'index de couleur CI 11725, CI 15510, CI 45370, CI 71105, pigments rouges avec les numéros d'index de couleur CI 12085, CI 12120, CI 12370, CI 12420, CI 12490, CI 14700, CI 15525, CI 15580, Cl 15620, CI 15630, CI 15800, CI 15850, Cl 15865, CI 15880, CI 17200, CI 26100, Cl 45380, Cl 45410, Cl 58000, Cl 73360, Cl 73915 et Cl 75470.

14. Procédé selon l'une des revendications 1 à 13, **caractérisé en ce que** le moyen (a) contient un ou plusieurs composés colorants (a3) choisis parmi le groupe des pigments à base d'une plaquette de substrat lamellaire, des pigments à base d'une plaquette de substrat lenticulaire et des pigments métallisés sous vide.

15. Procédé selon l'une des revendications 1 à 14, **caractérisé en ce que** le produit (a) contient au moins un support cosmétique (a4) choisi parmi le groupe constitué de poly-C₁ -C₆ - alkylèneglycols, le 1,2-propylèneglycol, le 1,3-propylèneglycol, le 1,2-butylèneglycol, le dipropylèneglycol, l'éthanol, l'isopropanol, le diéthylèneglycolmonoéthyléther, la glycérine, le phénoxyéthanol et alcool benzylique, de préférence tout particulièrement à partir de polyéthylèneglycols.

16. Agent selon l'une des revendications 1 à 15, **caractérisé en ce que** l'agent (a) contient, par rapport au poids total de l'agent (a), un ou plusieurs composants du support cosmétique (a4) en une quantité totale de 10,0 à 99,0 % en poids, de préférence de 30,0 à 99,0 % en poids, de préférence de 50,0 à 99,0 % en poids et de manière particulièrement préférée de 70,0 à 99,0 % en poids.

17. Procédé selon l'une des revendications 1 à 16, **caractérisé par** l'application du produit (b) dans les 10 minutes, de préférence dans les 5 minutes suivant l'application du produit (a).

18. Procédé selon l'une des revendications 1 à 17, **caractérisé par** l'
(2) application d'un agent (b) sur le matériau kératinique encore imprégné de l'agent (a) et la préparation d'un mélange des deux agents (a) et (b) sur le matériau kératinique par mélange manuel des deux agents.

19. Procédé selon l'une des revendications 1 à 18, **caractérisé en ce que** l'agent (b) - par rapport au poids total de l'agent (b) - contient 10 à 100 % en poids, de préférence 30 à 99,5 % en poids, de préférence 50 à 99 % en poids et de manière particulièrement préférée 80 à 99 % en poids d'eau.

20. Procédé selon l'une des revendications 1 à 19, **caractérisé en ce que** l'agent (b) contient au moins un agent épaississant.

21. Procédé selon l'une des revendications 1 à 20, **caractérisé en ce que** le rapport pondéral entre la quantité totale des composés organiques de silicium (a2) contenus dans le produit (a) et la quantité d'eau (b1) contenue dans le produit (b), c'est-à-dire que le rapport pondéral (a2)/(b1) est compris entre 1:100 et 1:1, de préférence entre 1:70 et 1:2, plus préférablement entre 1:70 et 1:5 et tout particulièrement préférablement entre 1:70 et 1:10.

22. Procédé selon l'une des revendications 1 à 21, **caractérisé par le fait que**
(3) action conjointe des deux agents (a) et (b) sur la matière kératinique pendant une durée comprise entre 10 secondes et 30 minutes, de préférence entre 30 secondes et 20, de préférence entre 1 minute et 15 minutes et tout particulièrement entre 5 minutes et 10 minutes.

23. Procédé selon l'une des revendications 1 à 22, **caractérisé par** le fait de
(4) rinçage des deux agents (a) et (b) sous l'eau courante.
